# EUROPEAN PATENT APPLICATION

(11) **EP 4 019 548 A1**
(43) Date of publication of application: **29.06.2022**
(21) Application number: 20020653.0
(22) Date of filing: 23.12.2020
(51) Int. Cl.: C07K 16/28

(54) **ANTI LAG3 VHHS AND THEIR USE**

(71) Applicant: Vrije Universiteit Brussel, 1090 Brussel (BE)
(72) Inventor: Breckpot, Karine, 1090 Brussel (BE); Broos, Katrijn, 1090 Brussel (BE); Devoogdt, Nick, 1090 Brussel (BE); Lecocq, Quentin, 1090 Brussel (BE); Keyaerts, Marleen, 1090 Brussel (BE)
(74) Representative: Arnold & Siedsma

(57) **Abstract**

The present disclosure is related to field of immune checkpoints in onco-immunology and there identification as potential targets for the treatment of cancer patients. Besides being considered as therapy targets, early detection of these immune checkpoints could equally be used in diagnosis, and monitoring of patients. Within said field, the present invention provides VHHs to detect and even quantify in a non-invasive way the immune checkpoint LAG-3, in particular the expression by tumor-infiltrating leukocytes and demonstrates how said detection is predictive in a response to combination therapy.

SEQUENCE LISTING

<110> Vrije Universiteit Brussel

<120> IMMUNE CHECKPOINTS

<130> VUB-022

<140> EP20020653.0
<141> 2020-12-23

<160> 129

<170> BiSSAP 1.3.6

<210> 1
<211> 8
<212> PRT
<213> Homo sapiens

<220>
<223> VHH ID 3185/CDR1

<400> 1
Gly Phe Thr Phe Asp Asp Tyr Ala
1               5

<210> 2
<211> 8
<212> PRT
<213> Homo sapiens

<220>
<223> VHH ID 3188/CRD1

<400> 2
Gly Phe Thr Phe Asp Asp Tyr Ala
1               5

<210> 3
<211> 8
<212> PRT
<213> Homo sapiens

<220>
<223> VHH ID 3176/ CDR1

<400> 3
Gly Phe Thr Phe Asp Asp Tyr Ala
1               5

<210> 4
<211> 8
<212> PRT
<213> Homo sapiens

<220>
<223> VHH ID 3150/CDR1

<400> 4
Gly Phe Thr Phe Asp Asp Tyr Ala
1               5

<210> 5
<211> 8
<212> PRT
<213> Homo sapiens

<220>
<223> VHH ID 3187 / CDR1

<400> 5
Gly Ile Thr Phe Asp Asp Tyr Ala
1               5

<210> 6
<211> 8
<212> PRT
<213> Homo sapiens

<220>
<223> VHH ID 3148 / CDR1

<400> 6
Gly Phe Ile Phe Asp Asp Tyr Ala
1               5

<210> 7
<211> 8
<212> PRT
<213> Homo sapiens

<220>
<223> VHH ID 3194 / CDR1

<400> 7
Gly Phe Thr Leu Asp Tyr Tyr Ala
1               5

<210> 8
<211> 8
<212> PRT
<213> Homo sapiens

<220>
<223> VHH ID 3195

<400> 8
Gly Phe Thr Leu Asp Tyr Tyr Ala
1               5

<210> 9
<211> 8
<212> PRT
<213> Homo sapiens

<220>

<223> VHH ID 3178 / CDR1

<400> 9
Gly Phe Thr Leu Asp Tyr Tyr Pro
1               5

<210> 10
<211> 8
<212> PRT
<213> Homo sapiens


<220>
<223> VHH ID 3183 / CDR1

<400> 10
Gly Phe Thr Leu Asp Gly Tyr Ala
1               5

<210> 11
<211> 8
<212> PRT
<213> Homo sapiens


<220>
<223> VHH ID 3197 / CDR1

<400> 11
Gly Phe Thr Leu Asp His Ala Ala
1               5

<210> 12
<211> 8
<212> PRT
<213> Homo sapiens


<220>
<223> VHH ID 3189 / CDR1

<400> 12
Gly Phe Thr Trp Asp Tyr Ser Ala
1               5

<210> 13
<211> 8
<212> PRT
<213> Homo sapiens


<220>
<223> VHH ID 3180 / CDR1

<400> 13
Gly Phe Ser Val Asp Asn Tyr Ala
1               5

<210> 14
<211> 8
<212> PRT
<213> Homo sapiens

<220>
<223> VHH ID 3169 / CDR1

<400> 14
Gly Leu Ser Leu Asp Tyr Tyr Ala
1               5

<210> 15
<211> 8
<212> PRT
<213> Homo sapiens


<220>
<223> VHH ID 3147 / CDR1

<400> 15
Gly Arg Thr Phe Gly Gly Tyr Asn
1               5

<210> 16
<211> 8
<212> PRT
<213> Homo sapiens


<220>
<223> VHH ID 3202 / CDR1

<400> 16
Gly Thr Ile Phe Ser Ile Asn Val
1               5

<210> 17
<211> 8
<212> PRT
<213> Homo sapiens


<220>
<223> VHH ID 3195 / CDR2

<400> 17
Ile Ser Ser Ser Asp Gly Ser Thr
1               5

<210> 18
<211> 8
<212> PRT
<213> Homo sapiens


<220>
<223> VHH ID 3196 / CDR2

<400> 18
Ile Ser Ser Ser Asp Gly Ser Thr
1               5

<210> 19
<211> 8
<212> PRT

<213> Homo sapiens

<220>
<223> VHH ID 3194 / CDR2

<400> 19
Ile Ser Ser Ser Asp Gly Ser Thr
1               5

<210> 20
<211> 8
<212> PRT
<213> Homo sapiens

<220>
<223> VHH ID 3185 / CDR2

<400> 20
Ile Ser Ser Ser Asp Gly Ser Thr
1               5

<210> 21
<211> 8
<212> PRT
<213> Homo sapiens

<220>
<223> VHH ID 3180 / CDR2

<400> 21
Ile Ser Ser Ser Asp Gly Ser Thr
1               5

<210> 22
<211> 8
<212> PRT
<213> Homo sapiens

<220>
<223> VHH ID 3178 / CDR2

<400> 22
Ile Ser Ser Ser Asp Gly Ser Thr
1               5

<210> 23
<211> 8
<212> PRT
<213> Homo sapiens

<220>
<223> VHH ID 3150 / CDR2

<400> 23
Ile Ser Ser Ser Asp Gly Ser Thr
1               5

<210> 24

<211> 8
<212> PRT
<213> Homo sapiens

<220>
<223> VHH ID 3188 / CDR2

<400> 24
Ile Arg Ser Ser Asp Gly Ser Thr
1               5

<210> 25
<211> 8
<212> PRT
<213> Homo sapiens

<220>
<223> VHH ID 3197 / CDR2

<400> 25
Ile Arg Ser Ser Asp Gly Ser Thr
1               5

<210> 26
<211> 8
<212> PRT
<213> Homo sapiens

<220>
<223> VHH ID 3187 / CDR2

<400> 26
Ile Arg Ser Ser Asp Asp Ser Thr
1               5

<210> 27
<211> 8
<212> PRT
<213> Homo sapiens

<220>
<223> VHH ID 3176 / CDR2

<400> 27
Met Arg Ser Ser Asp Gly Ser Thr
1               5

<210> 28
<211> 8
<212> PRT
<213> Homo sapiens

<220>
<223> VHH ID 3148 / CDR2

<400> 28
Ile Arg Ser Ser Asp Gly Asn Thr
1               5

<210> 29
<211> 8
<212> PRT
<213> Homo sapiens

<220>
<223> VHH ID 3183 / CDR2

<400> 29
Ile Ser Ser Ser Gly Gly Thr Thr
1               5

<210> 30
<211> 7
<212> PRT
<213> Homo sapiens

<220>
<223> VHH ID 3147 / CDR2

<400> 30
Ile Thr Gly Ser Gly Asp Ile
1               5

<210> 31
<211> 8
<212> PRT
<213> Homo sapiens

<220>
<223> VHH ID 3189 / CDR2

<400> 31
Ile Thr Ser Ser Gly Lys Asp Thr
1               5

<210> 32
<211> 7
<212> PRT
<213> Homo sapiens

<220>
<223> VHH ID 3202 / CDR2

<400> 32
Ile Thr Ser Arg Gly Ser Ala
1               5

<210> 33
<211> 14
<212> PRT
<213> Homo sapiens

<220>
<223> VHH ID 3194 / CDR3

<400> 33

Ala Thr Asp Arg Tyr Gly Thr Cys Thr Gly Pro Tyr Asp Asn
1               5                   10

<210> 34
<211> 14
<212> PRT
<213> Homo sapiens

<220>
<223> VHH ID 3195 / CD3

<400> 34
Ala Thr Asp His Tyr Gly Asp Cys Lys Gly Pro Tyr Asp Asn
1               5                   10

<210> 35
<211> 14
<212> PRT
<213> Homo sapiens

<220>
<223> VHH ID 3176 / CDR3

<400> 35
Ala Ser Asp Pro Trp Arg Glu Cys Lys Gly Pro Tyr Asp Ser
1               5                   10

<210> 36
<211> 14
<212> PRT
<213> Homo sapiens

<220>
<223> VHH ID 3180 / CDR3

<400> 36
Ala Arg Asp Pro Phe Gln Thr Cys Thr Gly Pro Tyr Asp Ser
1               5                   10

<210> 37
<211> 14
<212> PRT
<213> Homo sapiens

<220>
<223> VHH ID 3187 / CDR3

<400> 37
Ala Arg Asp Val Tyr Ser Asp Cys Lys Gly Pro Tyr Asp Ser
1               5                   10

<210> 38
<211> 14
<212> PRT
<213> Homo sapiens

<220>
<223> VHH ID 3148 / CDR3

<400> 38
Ala Thr Asp Ser Tyr Ser Asp Cys Lys Gly Pro Tyr Asp Tyr
1               5                   10

<210> 39
<211> 14
<212> PRT
<213> Homo sapiens

<220>
<223> VHH ID 3183 / CDR3

<400> 39
Ala Ala Asp Ser Tyr Gly Asp Cys Lys Gly Pro Tyr Asp Tyr
1               5                   10

<210> 40
<211> 14
<212> PRT
<213> Homo sapiens

<220>
<223> VHH ID 3150 / CDR3

<400> 40
Ala Ala Asp Arg Tyr Arg Asp Cys Arg Gly Pro Tyr Asp Tyr
1               5                   10

<210> 41
<211> 14
<212> PRT
<213> Homo sapiens

<220>
<223> VHH ID 3188 / CDR3

<400> 41
Ala Ala Asp Arg Tyr Arg Asp Cys Arg Gly Pro Tyr Asp Tyr
1               5                   10

<210> 42
<211> 14
<212> PRT
<213> Homo sapiens

<220>
<223> VHH ID 3197 / CDR3

<400> 42
Ala Thr Asp Lys Phe Ala Asp Cys Lys Gly Pro Tyr Asp Tyr
1               5                   10

<210> 43
<211> 14
<212> PRT
<213> Homo sapiens

<220>
<223> VHH ID 3196 / CDR3

<400> 43
Ala Thr Asp Pro Tyr Asn Ser Cys Thr Gly Pro Tyr Asn Tyr
1               5                   10

<210> 44
<211> 15
<212> PRT
<213> Homo sapiens


<220>
<223> VHH ID 3178 / CDR3

<400> 44
Ala Ala Gly Pro Ser Gly Tyr Gly Cys Asn Thr Arg Trp Asp Tyr
1               5                   10                  15

<210> 45
<211> 16
<212> PRT
<213> Homo sapiens


<220>
<223> VHH ID 3189 / CDR3

<400> 45
Ala Ala Pro Gly Arg Val Arg Gly Ser Cys Asn Gly Arg Trp Asp Tyr
1               5                   10                  15



<210> 46
<211> 14
<212> PRT
<213> Homo sapiens


<220>
<223> VHH ID 3185 / CDR3

<400> 46
Ala Ala Arg Glu Gly Asp Ser Cys Ser Gly Arg Trp Asp Tyr
1               5                   10

<210> 47
<211> 14
<212> PRT
<213> Homo sapiens


<220>
<223> VHH ID 3202 / CDR3

<400> 47
Asn Ala Leu Ala Val Gln Thr Ala Asp Gly Val Ser Asp Tyr
1               5                   10

<210> 48
<211> 20

<212> PRT
<213> Homo sapiens

<220>
<223> VHH ID 3147 / CDR3

<400> 48
Ala Ser Ser Ser Arg Phe Ser Gly Thr Leu Trp Ala Gly Gln Thr Pro
1               5                   10                  15
Asn Tyr Asp Tyr
            20

<210> 49
<211> 25
<212> PRT
<213> Homo sapiens

<220>
<223> VHH ID 3188 / FR1

<400> 49
Gln Val Gln Leu Gln Glu Ser Gly Gly Gly Leu Val His Pro Gly Gly
1               5                   10                  15
Ser Leu Arg Leu Ser Cys Ala Ala Ser
            20                  25

<210> 50
<211> 25
<212> PRT
<213> Homo sapiens

<220>
<223> VHH ID 3189 / FR1

<400> 50
Gln Val Gln Leu Gln Glu Ser Gly Gly Gly Leu Val Gln Pro Gly Gly
1               5                   10                  15
Ser Leu Lys Leu Ser Cys Ala Ala Ser
            20                  25

<210> 51
<211> 25
<212> PRT
<213> Homo sapiens

<220>
<223> VHH ID 3194 / FR1

<400> 51
Gln Val Gln Leu Gln Glu Ser Gly Gly Gly Leu Val Gln Pro Gly Gly
1               5                   10                  15
Ser Leu Arg Leu Ser Cys Ala Ala Ser
            20                  25

<210> 52
<211> 25
<212> PRT
<213> Homo sapiens

<220>
<223> VHH ID 3183 / FR1

<400> 52
Gln Val Gln Leu Gln Glu Ser Gly Gly Gly Leu Val Gln Pro Gly Gly
1               5                   10                  15
Ser Leu Arg Leu Ser Cys Glu Ala Ser
            20                  25

<210> 53
<211> 25
<212> PRT
<213> Homo sapiens


<220>
<223> VHH ID 3180 / FR1

<400> 53
Gln Val Gln Leu Gln Glu Ser Gly Gly Gly Leu Val Gln Pro Gly Gly
1               5                   10                  15
Ser Leu Arg Leu Ser Cys Ala Ala Ser
            20                  25

<210> 54
<211> 25
<212> PRT
<213> Homo sapiens


<220>
<223> VHH ID 3196 / FR1

<400> 54
Gln Val Gln Leu Gln Glu Ser Gly Gly Gly Leu Val Gln Pro Gly Gly
1               5                   10                  15
Ser Leu Arg Leu Ser Cys Ala Ala Ser
            20                  25

<210> 55
<211> 25
<212> PRT
<213> Homo sapiens


<220>
<223> VHH ID 3178 / FR1

<400> 55
Gln Val Gln Leu Gln Glu Ser Gly Gly Gly Leu Val Gln Pro Gly Gly
1               5                   10                  15
Ser Leu Arg Leu Ser Cys Ala Ala Ser
            20                  25

<210> 56
<211> 25
<212> PRT
<213> Homo sapiens


<220>
<223> VHH ID 3195 / FR1

<400> 56
Gln Val Gln Leu Gln Glu Ser Gly Gly Ser Leu Val Gln Pro Gly Gly
1               5                   10                  15
Ser Leu Arg Leu Ser Cys Ala Ala Ser
            20                  25

<210> 57
<211> 25
<212> PRT
<213> Homo sapiens

<220>
<223> VHH ID 3197 / FR1

<400> 57
Gln Val Gln Leu Gln Glu Ser Gly Gly Asp Ser Val Gln Pro Gly Gly
1               5                   10                  15
Ser Leu Arg Leu Ser Cys Ala Ala Ser
            20                  25

<210> 58
<211> 25
<212> PRT
<213> Homo sapiens

<220>
<223> VHH ID 3147 / FR1

<400> 58
Gln Val Gln Leu Gln Glu Ser Gly Gly Gly Leu Val Gln Ala Gly Gly
1               5                   10                  15
Ser Leu Arg Leu Ser Cys Ala Thr Ser
            20                  25

<210> 59
<211> 25
<212> PRT
<213> Homo sapiens

<220>
<223> VHH ID 3148 / FR1

<400> 59
Gln Val Gln Leu Gln Glu Ser Gly Gly Gly Leu Val Gln Ala Gly Gly
1               5                   10                  15
Ser Leu Arg Leu Ser Cys Ala Ala Ser
            20                  25

<210> 60
<211> 25
<212> PRT
<213> Homo sapiens

<220>
<223> VHH ID 3150 / FR1

<400> 60
Gln Val Gln Leu Val Glu Ser Gly Gly Gly Leu Val Gln Ala Gly Glu

1                   5                   10                  15
Ser Leu Arg Leu Ser Cys Ala Ala Ser
            20                  25

<210> 61
<211> 25
<212> PRT
<213> Homo sapiens

<220>
<223> VHH ID 3176 / FR1

<400> 61
Gln Val Gln Leu Gln Glu Ser Gly Gly Gly Leu Val Gln Ala Gly Gly
1               5                   10                  15
Ser Leu Arg Leu Ser Cys Ala Ala Ser
            20                  25

<210> 62
<211> 25
<212> PRT
<213> Homo sapiens

<220>
<223> VHH ID 3185 / FR1

<400> 62
Gln Val Gln Leu Gln Glu Ser Gly Gly Gly Leu Val Gln Ala Gly Gly
1               5                   10                  15
Ser Leu Arg Leu Ser Cys Ala Ala Ser
            20                  25

<210> 63
<211> 25
<212> PRT
<213> Homo sapiens

<220>
<223> VHH ID 3187 / FR1

<400> 63
Gln Val Gln Leu Gln Glu Ser Gly Gly Gly Leu Val Gln Ala Gly Gly
1               5                   10                  15
Ser Leu Arg Leu Ser Cys Ala Ala Ser
            20                  25

<210> 64
<211> 25
<212> PRT
<213> Homo sapiens

<220>
<223> VHH ID 3202 / FR1

<400> 64
Gln Val Gln Leu Gln Glu Ser Gly Gly Gly Leu Val Gln Ala Gly Gly
1               5                   10                  15
Ser Leu Arg Leu Ser Cys Ala Ala Ser
            20                  25

<210> 65
<211> 17
<212> PRT
<213> Homo sapiens

<220>
<223> VHH ID 3196 / FR2

<400> 65
Ile Gly Trp Phe Arg Gln Ala Pro Gly Lys Glu Arg Glu Gly Val Ser
1               5                   10                  15
Cys

<210> 66
<211> 17
<212> PRT
<213> Homo sapiens

<220>
<223> VHH ID 3197 / FR2

<400> 66
Ile Gly Trp Phe Arg Gln Ala Pro Gly Lys Glu Arg Glu Gly Val Ser
1               5                   10                  15
Cys

<210> 67
<211> 17
<212> PRT
<213> Homo sapiens

<220>
<223> VHH ID 3195 / FR2

<400> 67
Ile Gly Trp Phe Arg Gln Ala Pro Gly Lys Glu Arg Glu Gly Val Ser
1               5                   10                  15
Cys

<210> 68
<211> 17
<212> PRT
<213> Homo sapiens

<220>
<223> VHH ID 3194 / FR2

<400> 68
Ile Gly Trp Phe Arg Gln Ala Pro Gly Lys Glu Arg Glu Gly Val Ser
1               5                   10                  15
Cys

<210> 69
<211> 17

<212> PRT
<213> Homo sapiens

<220>
<223> VHH ID 3189 / FR2

<400> 69
Ile Gly Trp Phe Arg Gln Ala Pro Gly Lys Glu Arg Glu Gly Val Ser
1               5                   10                  15
Cys

<210> 70
<211> 17
<212> PRT
<213> Homo sapiens

<220>
<223> VHH ID 3188 / FR2

<400> 70
Ile Gly Trp Phe Arg Gln Ala Pro Gly Lys Glu Arg Glu Gly Val Ser
1               5                   10                  15
Cys

<210> 71
<211> 17
<212> PRT
<213> Homo sapiens

<220>
<223> VHH ID 3187 / FR2

<400> 71
Ile Gly Trp Phe Arg Gln Ala Pro Gly Lys Glu Arg Glu Gly Val Ser
1               5                   10                  15
Cys

<210> 72
<211> 17
<212> PRT
<213> Homo sapiens

<220>
<223> VHH ID 3185 / FR2

<400> 72
Ile Gly Trp Phe Arg Gln Ala Pro Gly Lys Glu Arg Glu Gly Val Ser
1               5                   10                  15
Cys

<210> 73
<211> 17
<212> PRT
<213> Homo sapiens

<220>
<223> VHH ID 3178 / FR2

<400> 73
Ile Gly Trp Phe Arg Gln Ala Pro Gly Lys Glu Arg Glu Gly Val Ser
1               5                   10                  15
Cys


<210> 74
<211> 17
<212> PRT
<213> Homo sapiens


<220>
<223> VHH ID 3176 / FR2

<400> 74
Ile Gly Trp Phe Arg Gln Ala Pro Gly Lys Glu Arg Glu Gly Val Ser
1               5                   10                  15
Cys


<210> 75
<211> 17
<212> PRT
<213> Homo sapiens


<220>
<223> VHH ID 3150 / FR2

<400> 75
Ile Gly Trp Phe Arg Gln Ala Pro Gly Lys Glu Arg Glu Gly Val Ser
1               5                   10                  15
Cys


<210> 76
<211> 17
<212> PRT
<213> Homo sapiens


<220>
<223> VHH ID 3148 / FR2

<400> 76
Ile Gly Trp Phe Arg Gln Ala Pro Gly Lys Glu Arg Glu Gly Val Ser
1               5                   10                  15
Cys


<210> 77
<211> 17
<212> PRT
<213> Homo sapiens


<220>
<223> VHH ID 3180 / FR2

<400> 77
Ile Gly Trp Phe Arg Gln Ala Pro Gly Lys Gln Arg Glu Gly Val Ser
1               5                   10                  15
Cys

<210> 78
<211> 17
<212> PRT
<213> Homo sapiens

<220>
<223> VHH ID 3183 / FR2

<400> 78
Ile Gly Ala Phe Arg Gln Ala Pro Gly Lys Glu Arg Glu Ala Val Ser
1               5                   10                  15
Cys

<210> 79
<211> 17
<212> PRT
<213> Homo sapiens

<220>
<223> VHH ID 3147 / FR2

<400> 79
Arg Gly Trp Phe Arg Gln Thr Gln Gly Lys Glu Arg Glu Phe Val Ala
1               5                   10                  15
Ala

<210> 80
<211> 17
<212> PRT
<213> Homo sapiens

<220>
<223> VHH ID 3202 / FR2

<400> 80
Met Gly Trp Tyr Arg Gln Ala Leu Gly Lys Gln Arg Glu Leu Val Ala
1               5                   10                  15
Leu

<210> 81
<211> 38
<212> PRT
<213> Homo sapiens

<220>
<223> VHH ID 3150 / FR3

<400> 81
Tyr Tyr Ala Asp Ser Val Lys Gly Arg Phe Thr Ile Ser Ser Asp Asn

1                   5                   10                  15
Ala Glu Asn Thr Val Tyr Leu Gln Met Asn Ser Leu Lys Pro Glu Asp
            20                  25                  30
Thr Ala Val Tyr Tyr Cys
        35

<210> 82
<211> 38
<212> PRT
<213> Homo sapiens


<220>
<223> VHH ID 3188 / FR3

<400> 82
Tyr Tyr Ala Asp Ser Val Lys Gly Arg Phe Thr Ile Ser Ser Asp Asn
1                   5                   10                  15
Ala Glu Asn Thr Val Tyr Leu Gln Met Asn Ser Leu Lys Pro Glu Asp
            20                  25                  30
Thr Ala Val Tyr Tyr Cys
        35

<210> 83
<211> 38
<212> PRT
<213> Homo sapiens


<220>
<223> VHH ID 3187 / FR3

<400> 83
Tyr Tyr Ala Asp Ser Val Lys Gly Arg Phe Thr Ile Ser Ser Asp Asn
1                   5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 84
<211> 38
<212> PRT
<213> Homo sapiens


<220>
<223> VHH ID 3148 / FR3

<400> 84
Tyr Tyr Ala Asp Ser Val Lys Gly Arg Phe Thr Ile Ser Ser Asp Asn
1                   5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 85
<211> 38
<212> PRT
<213> Homo sapiens

<220>
<223> VHH ID 3176 / FR3

<400> 85
Tyr Tyr Ala Asp Ser Val Lys Gly Arg Phe Thr Ile Ser Ser Asp Asn
1               5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 86
<211> 38
<212> PRT
<213> Homo sapiens


<220>
<223> VHH ID 3185 / FR3

<400> 86
Arg Tyr Ala Asp Ser Val Lys Gly Arg Phe Thr Ile Ser Ser Asp Asn
1               5                   10                  15
Ala Lys Asn Thr Val Arg Leu Gln Met Asp Ser Leu Lys Pro Glu Asp
            20                  25                  30
Thr Ala Val Tyr Tyr Cys
        35

<210> 87
<211> 38
<212> PRT
<213> Homo sapiens


<220>
<223> VHH ID 3197 / FR3

<400> 87
Tyr Tyr Ala Asp Ser Val Lys Gly Arg Phe Thr Ile Ser Lys Asp Asn
1               5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 88
<211> 38
<212> PRT
<213> Homo sapiens


<220>
<223> VHH ID 3202 / FR3

<400> 88
His Leu Ala Asp Ser Val Lys Gly Arg Phe Thr Ile Ser Gly Asp Asn
1               5                   10                  15
Ala Lys Asn Thr Val Tyr Leu Gln Met Asn Ser Leu Lys Ala Glu Asp
            20                  25                  30
Thr Ala Val Tyr Tyr Cys
        35

<210> 89

<211> 38
<212> PRT
<213> Homo sapiens

<220>
<223> VHH ID 3194 / FR3

<400> 89
Tyr Tyr Ala Asp Ser Val Lys Gly Arg Phe Thr Ile Ser Arg Asp Asn
1               5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 90
<211> 38
<212> PRT
<213> Homo sapiens

<220>
<223> VHH ID 3178 / FR3

<400> 90
Arg Tyr Ala Asp Ser Val Lys Gly Arg Phe Thr Ile Ser Arg Asp Asn
1               5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 91
<211> 38
<212> PRT
<213> Homo sapiens

<220>
<223> VHH ID3196 / FR3

<400> 91
Arg Tyr Ala Asp Ser Val Lys Gly Arg Phe Thr Ile Ser Arg Asp Asn
1               5                   10                  15
Ala Lys Asn Thr Val Tyr Leu Gln Met Asn Ser Leu Lys Pro Glu Asp
            20                  25                  30
Thr Ala Val Tyr Tyr Cys
        35

<210> 92
<211> 38
<212> PRT
<213> Homo sapiens

<220>
<223> VHH ID 3180 / FR3

<400> 92
Lys Tyr Ala Asp Ser Val Lys Gly Arg Phe Thr Ile Ser Arg Asp Asn
1               5                   10                  15
Ala Lys Asn Thr Val Tyr Leu Gln Met Asn Ser Leu Lys Pro Glu Asp

                20                  25                  30
Thr Ala Val Tyr Tyr Cys
            35

<210> 93
<211> 38
<212> PRT
<213> Homo sapiens



<220>
<223> VHH ID 3183 / FR3

<400> 93
Tyr Tyr Ala Asp Ser Val Lys Gly Arg Phe Thr Ile Ser Arg Asp Asn
1               5                   10                  15
Ala Lys Asn Met Val Tyr Leu Gln Met Asn Ser Leu Lys Pro Glu Asp
            20                  25                  30
Thr Ala Val Tyr Tyr Cys
            35

<210> 94
<211> 38
<212> PRT
<213> Homo sapiens



<220>
<223> VHH ID 3189 / FR3

<400> 94
Gln Tyr Leu Asp Ser Val Lys Gly Arg Phe Thr Ile Ser Arg Asp Asn
1               5                   10                  15
Thr Lys Asn Thr Val Tyr Leu Gln Met Asn Asn Leu Lys Pro Glu Asp
            20                  25                  30
Thr Ala Val Tyr Tyr Cys
            35

<210> 95
<211> 38
<212> PRT
<213> Homo sapiens



<220>
<223> VHH ID 3195 / FR3

<400> 95
Tyr Tyr Ala Asp Ser Val Lys Gly Arg Phe Ala Ile Ser Arg Asp Asn
1               5                   10                  15
Val Lys Asn Thr Val Phe Leu Gln Met Asn Ser Leu Lys Pro Glu Asp
            20                  25                  30
Thr Ala Val Tyr Tyr Cys
            35

<210> 96
<211> 38
<212> PRT
<213> Homo sapiens



<220>
<223> VHH ID 3147 / FR3

<400> 96
Thr Tyr Ala Asp Ser Val Lys Gly Arg Phe Thr Ile Gly Arg Asp Asn
1               5                   10                  15
Thr Lys Asn Met Val Phe Leu Gln Met Asp Ser Leu Glu Pro Glu Asp
            20                  25                  30
Thr Ala Val Tyr Leu Cys
        35

<210> 97
<211> 11
<212> PRT
<213> Homo sapiens

<220>
<223> VHH ID 3197 / FR4

<400> 97
Trp Gly Gln Gly Thr Gln Val Thr Val Ser Ser
1               5                   10

<210> 98
<211> 11
<212> PRT
<213> Homo sapiens

<220>
<223> VHH ID 3202 / FR4

<400> 98
Trp Gly Gln Gly Thr Gln Val Thr Val Ser Ser
1               5                   10

<210> 99
<211> 11
<212> PRT
<213> Homo sapiens

<220>
<223> VHH ID 3195 / FR4

<400> 99
Trp Gly Gln Gly Thr Gln Val Thr Val Ser Ser
1               5                   10

<210> 100
<211> 11
<212> PRT
<213> Homo sapiens

<220>
<223> VHH ID 3196 / FR4

<400> 100
Trp Gly Gln Gly Thr Gln Val Thr Val Ser Ser
1               5                   10

<210> 101
<211> 11

<212> PRT
<213> Homo sapiens

<220>
<223> VHH ID 3194 / FR4

<400> 101
Trp Gly Gln Gly Thr Gln Val Thr Val Ser Ser
1               5                   10

<210> 102
<211> 11
<212> PRT
<213> Homo sapiens

<220>
<223> VHH ID 3189 / FR4

<400> 102
Trp Gly Gln Gly Thr Gln Val Thr Val Ser Ser
1               5                   10

<210> 103
<211> 11
<212> PRT
<213> Homo sapiens

<220>
<223> VHH ID 3188 / FR4

<400> 103
Trp Gly Gln Gly Thr Gln Val Thr Val Ser Ser
1               5                   10

<210> 104
<211> 11
<212> PRT
<213> Homo sapiens

<220>
<223> VHH ID 3185 / FR4

<400> 104
Trp Gly Gln Gly Thr Gln Val Thr Val Ser Ser
1               5                   10

<210> 105
<211> 11
<212> PRT
<213> Homo sapiens

<220>
<223> VHH ID 3183 / FR4

<400> 105
Trp Gly Gln Gly Thr Gln Val Thr Val Ser Ser
1               5                   10

<210> 106
<211> 11
<212> PRT
<213> Homo sapiens

<220>
<223> VHH ID 3180 / FR4

<400> 106
Trp Gly Gln Gly Thr Gln Val Thr Val Ser Ser
1               5                   10

<210> 107
<211> 11
<212> PRT
<213> Homo sapiens

<220>
<223> VHH ID 3178 / FR4

<400> 107
Trp Gly Gln Gly Thr Gln Val Thr Val Ser Ser
1               5                   10

<210> 108
<211> 11
<212> PRT
<213> Homo sapiens

<220>
<223> VHH ID 3150 / FR4

<400> 108
Trp Gly Gln Gly Thr Gln Val Thr Val Ser Ser
1               5                   10

<210> 109
<211> 11
<212> PRT
<213> Homo sapiens

<220>
<223> VHH ID 3148 / FR4

<400> 109
Trp Gly Gln Gly Thr Gln Val Thr Val Ser Ser
1               5                   10

<210> 110
<211> 11
<212> PRT
<213> Homo sapiens

<220>
<223> VHH ID 3147 / FR4

<400> 110
Trp Gly Gln Gly Thr Gln Val Thr Val Ser Ser

1                   5                   10

<210> 111
<211> 11
<212> PRT
<213> Homo sapiens

<220>
<223> VHH ID 3176 / FR4

<400> 111
Trp Gly Arg Gly Thr Gln Val Thr Val Ser Ser
1               5                   10

<210> 112
<211> 11
<212> PRT
<213> Homo sapiens

<220>
<223> VHH ID 3187 / FR4

<400> 112
Trp Gly Arg Gly Thr Gln Val Thr Val Ser Ser
1               5                   10

<210> 113
<211> 121
<212> PRT
<213> Homo sapiens

<220>
<223> Nb 3150

<400> 113
Gln Val Gln Leu Gln Glu Ser Gly Gly Gly Leu Val Gln Ala Gly Gly
1               5                   10                  15
Ser Leu Arg Leu Ser Cys Ala Ala Ser Gly Ile Thr Phe Asp Asp Tyr
            20                  25                  30
Ala Ile Gly Trp Phe Arg Gln Ala Pro Gly Lys Glu Arg Glu Gly Val
        35                  40                  45
Ser Cys Ile Arg Ser Ser Asp Asp Ser Thr Tyr Tyr Ala Asp Ser Val
    50                  55                  60
Lys Gly Arg Phe Thr Ile Ser Ser Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Arg Asp Val Tyr Ser Asp Cys Lys Gly Pro Tyr Asp Ser Trp Gly
            100                 105                 110
Arg Gly Thr Gln Val Thr Val Ser Ser
        115                 120

<210> 114
<211> 121
<212> PRT
<213> Homo sapiens

<220>
<223> Nb 3148

<400> 114
Gln Val Gln Leu Gln Glu Ser Gly Gly Gly Leu Val Gln Ala Gly Gly
1               5                   10                  15
Ser Leu Arg Leu Ser Cys Ala Ala Ser Gly Phe Ile Phe Asp Asp Tyr
            20                  25                  30
Ala Ile Gly Trp Phe Arg Gln Ala Pro Gly Lys Glu Arg Glu Gly Val
        35                  40                  45
Ser Cys Ile Arg Ser Ser Asp Gly Asn Thr Tyr Tyr Ala Asp Ser Val
    50                  55                  60
Lys Gly Arg Phe Thr Ile Ser Ser Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Thr Asp Ser Tyr Ser Asp Cys Lys Gly Pro Tyr Asp Tyr Trp Gly
            100                 105                 110
Gln Gly Thr Gln Val Thr Val Ser Ser
        115                 120

<210> 115
<211> 121
<212> PRT
<213> Homo sapiens

<220>
<223> Nb 3183

<400> 115
Gln Val Gln Leu Gln Glu Ser Gly Gly Gly Leu Val Gln Pro Gly Gly
1               5                   10                  15
Ser Leu Arg Leu Ser Cys Glu Ala Ser Gly Phe Thr Leu Asp Gly Tyr
            20                  25                  30
Ala Ile Gly Ala Phe Arg Gln Ala Pro Gly Lys Glu Arg Glu Ala Val
        35                  40                  45
Ser Cys Ile Ser Ser Ser Gly Gly Thr Thr Tyr Tyr Ala Asp Ser Val
    50                  55                  60
Lys Gly Arg Phe Thr Ile Ser Arg Asp Asn Ala Lys Asn Met Val Tyr
65                  70                  75                  80
Leu Gln Met Asn Ser Leu Lys Pro Glu Asp Thr Ala Val Tyr Tyr Cys
                85                  90                  95
Ala Ala Asp Ser Tyr Gly Asp Cys Lys Gly Pro Tyr Asp Tyr Trp Gly
            100                 105                 110
Gln Gly Thr Gln Val Thr Val Ser Ser
        115                 120

<210> 116
<211> 121
<212> PRT
<213> Homo sapiens

<220>
<223> Nb 3195

<400> 116
Gln Val Gln Leu Gln Glu Ser Gly Gly Ser Leu Val Gln Pro Gly Gly
1               5                   10                  15
Ser Leu Arg Leu Ser Cys Ala Ala Ser Gly Phe Thr Leu Asp Tyr Tyr
            20                  25                  30
Ala Ile Gly Trp Phe Arg Gln Ala Pro Gly Lys Glu Arg Glu Gly Val
        35                  40                  45
Ser Cys Ile Ser Ser Ser Asp Gly Ser Thr Tyr Tyr Ala Asp Ser Val

50                  55                  60
Lys Gly Arg Phe Ala Ile Ser Arg Asp Asn Val Lys Asn Thr Val Phe
65                  70                  75                  80
Leu Gln Met Asn Ser Leu Lys Pro Glu Asp Thr Ala Val Tyr Tyr Cys
                85                  90                  95
Ala Thr Asp His Tyr Gly Asp Cys Lys Gly Pro Tyr Asp Asn Trp Gly
            100                 105                 110
Gln Gly Thr Gln Val Thr Val Ser Ser
        115                 120

<210> 117
<211> 121
<212> PRT
<213> Homo sapiens

<220>
<223> Nb 3180

<400> 117
Gln Val Gln Leu Gln Glu Ser Gly Gly Gly Leu Val Gln Pro Gly Gly
1               5                   10                  15
Ser Leu Arg Leu Ser Cys Ala Ala Ser Gly Phe Ser Val Asp Asn Tyr
            20                  25                  30
Ala Ile Gly Trp Phe Arg Gln Ala Pro Gly Lys Gln Arg Glu Gly Val
        35                  40                  45
Ser Cys Ile Ser Ser Ser Asp Gly Ser Thr Lys Tyr Ala Asp Ser Val
    50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Arg Asp Pro Phe Gln Thr Cys Thr Gly Pro Tyr Asp Ser Trp Gly
            100                 105                 110
Gln Gly Thr Gln Val Thr Val Ser Ser
        115                 120

<210> 118
<211> 120
<212> PRT
<213> Homo sapiens

<220>
<223> Nb 3202

<400> 118
Gln Val Gln Leu Gln Glu Ser Gly Gly Gly Leu Val Gln Ala Gly Gly
1               5                   10                  15
Ser Leu Arg Leu Ser Cys Ala Ala Ser Gly Thr Ile Phe Ser Ile Asn
            20                  25                  30
Val Met Gly Trp Tyr Arg Gln Ala Leu Gly Lys Gln Arg Glu Leu Val
        35                  40                  45
Ala Leu Ile Thr Ser Arg Gly Ser Ala His Leu Ala Asp Ser Val Lys
    50                  55                  60
Gly Arg Phe Thr Ile Ser Gly Asp Asn Ala Lys Asn Thr Val Tyr Leu
65                  70                  75                  80
Gln Met Asn Ser Leu Lys Ala Glu Asp Thr Ala Val Tyr Tyr Cys Asn
                85                  90                  95
Ala Leu Ala Val Gln Thr Ala Asp Gly Val Ser Asp Tyr Trp Gly Gln
            100                 105                 110
Gly Thr Gln Val Thr Val Ser Ser
        115                 120

<210> 119
<211> 121
<212> PRT
<213> Homo sapiens

<220>
<223> Nb 3150

<400> 119
Gln Val Gln Leu Val Glu Ser Gly Gly Gly Leu Val Gln Ala Gly Glu
1               5                   10                  15
Ser Leu Arg Leu Ser Cys Ala Ala Ser Gly Phe Thr Phe Asp Asp Tyr
            20                  25                  30
Ala Ile Gly Trp Phe Arg Gln Ala Pro Gly Lys Glu Arg Glu Gly Val
        35                  40                  45
Ser Cys Ile Ser Ser Ser Asp Gly Ser Thr Tyr Tyr Ala Asp Ser Val
    50                  55                  60
Lys Gly Arg Phe Thr Ile Ser Ser Asp Asn Ala Glu Asn Thr Val Tyr
65                  70                  75                  80
Leu Gln Met Asn Ser Leu Lys Pro Glu Asp Thr Ala Val Tyr Tyr Cys
                85                  90                  95
Ala Ala Asp Arg Tyr Arg Asp Cys Arg Gly Pro Tyr Asp Tyr Trp Gly
            100                 105                 110
Gln Gly Thr Gln Val Thr Val Ser Ser
        115                 120

<210> 120
<211> 121
<212> PRT
<213> Homo sapiens

<220>
<223> Nb 3176

<400> 120
Gln Val Gln Leu Gln Glu Ser Gly Gly Gly Leu Val Gln Ala Gly Gly
1               5                   10                  15
Ser Leu Arg Leu Ser Cys Ala Ala Ser Gly Phe Thr Phe Asp Asp Tyr
            20                  25                  30
Ala Ile Gly Trp Phe Arg Gln Ala Pro Gly Lys Glu Arg Glu Gly Val
        35                  40                  45
Ser Cys Met Arg Ser Ser Asp Gly Ser Thr Tyr Tyr Ala Asp Ser Val
    50                  55                  60
Lys Gly Arg Phe Thr Ile Ser Ser Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Ser Asp Pro Trp Arg Glu Cys Lys Gly Pro Tyr Asp Ser Trp Gly
            100                 105                 110
Arg Gly Thr Gln Val Thr Val Ser Ser
        115                 120

<210> 121
<211> 121
<212> PRT
<213> Homo sapiens

<220>
<223> Nb 3188

<400> 121
Gln Val Gln Leu Gln Glu Ser Gly Gly Gly Leu Val His Pro Gly Gly
1               5                   10                  15
Ser Leu Arg Leu Ser Cys Ala Ala Ser Gly Phe Thr Phe Asp Asp Tyr
            20                  25                  30
Ala Ile Gly Trp Phe Arg Gln Ala Pro Gly Lys Glu Arg Glu Gly Val
        35                  40                  45
Ser Cys Ile Arg Ser Ser Asp Gly Ser Thr Tyr Tyr Ala Asp Ser Val
    50                  55                  60
Lys Gly Arg Phe Thr Ile Ser Ser Asp Asn Ala Glu Asn Thr Val Tyr
65                  70                  75                  80
Leu Gln Met Asn Ser Leu Lys Pro Glu Asp Thr Ala Val Tyr Tyr Cys
                85                  90                  95
Ala Ala Asp Arg Tyr Arg Asp Cys Arg Gly Pro Tyr Asp Tyr Trp Gly
            100                 105                 110
Gln Gly Thr Gln Val Thr Val Ser Ser
        115                 120

<210> 122
<211> 121
<212> PRT
<213> Homo sapiens

<220>
<223> Nb 3194

<400> 122
Gln Val Gln Leu Gln Glu Ser Gly Gly Gly Leu Val Gln Pro Gly Gly
1               5                   10                  15
Ser Leu Arg Leu Ser Cys Ala Ala Ser Gly Phe Thr Leu Asp Tyr Tyr
            20                  25                  30
Ala Ile Gly Trp Phe Arg Gln Ala Pro Gly Lys Glu Arg Glu Gly Val
        35                  40                  45
Ser Cys Ile Ser Ser Ser Asp Gly Ser Thr Tyr Tyr Ala Asp Ser Val
    50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Thr Asp Arg Tyr Gly Thr Cys Thr Gly Pro Tyr Asp Asn Trp Gly
            100                 105                 110
Gln Gly Thr Gln Val Thr Val Ser Ser
        115                 120

<210> 123
<211> 121
<212> PRT
<213> Homo sapiens

<220>
<223> Nb 3185

<400> 123
Gln Val Gln Leu Gln Glu Ser Gly Gly Gly Leu Val Gln Ala Gly Gly
1               5                   10                  15
Ser Leu Arg Leu Ser Cys Ala Ala Ser Gly Phe Thr Phe Asp Asp Tyr
            20                  25                  30
Ala Ile Gly Trp Phe Arg Gln Ala Pro Gly Lys Glu Arg Glu Gly Val
        35                  40                  45
Ser Cys Ile Ser Ser Ser Asp Gly Ser Thr Arg Tyr Ala Asp Ser Val

50                  55                  60
Lys Gly Arg Phe Thr Ile Ser Ser Asp Asn Ala Lys Asn Thr Val Arg
65                  70                  75                  80
Leu Gln Met Asp Ser Leu Lys Pro Glu Asp Thr Ala Val Tyr Tyr Cys
                85                  90                  95
Ala Ala Arg Glu Gly Asp Ser Cys Ser Gly Arg Trp Asp Tyr Trp Gly
            100                 105                 110
Gln Gly Thr Gln Val Thr Val Ser Ser
        115                 120

<210> 124
<211> 121
<212> PRT
<213> Homo sapiens

<220>
<223> Nb 3197

<400> 124
Gln Val Gln Leu Gln Glu Ser Gly Gly Asp Ser Val Gln Pro Gly Gly
1               5                   10                  15
Ser Leu Arg Leu Ser Cys Ala Ala Ser Gly Phe Thr Leu Asp His Ala
            20                  25                  30
Ala Ile Gly Trp Phe Arg Gln Ala Pro Gly Lys Glu Arg Glu Gly Val
        35                  40                  45
Ser Cys Ile Arg Ser Ser Asp Gly Ser Thr Tyr Tyr Ala Asp Ser Val
    50                  55                  60
Lys Gly Arg Phe Thr Ile Ser Lys Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Thr Asp Lys Phe Ala Asp Cys Lys Gly Pro Tyr Asp Tyr Trp Gly
            100                 105                 110
Gln Gly Thr Gln Val Thr Val Ser Ser
        115                 120

<210> 125
<211> 123
<212> PRT
<213> Homo sapiens

<220>
<223> Nb 3189

<400> 125
Gln Val Gln Leu Gln Glu Ser Gly Gly Gly Leu Val Gln Pro Gly Gly
1               5                   10                  15
Ser Leu Lys Leu Ser Cys Ala Ala Ser Gly Phe Thr Trp Asp Tyr Ser
            20                  25                  30
Ala Ile Gly Trp Phe Arg Gln Ala Pro Gly Lys Glu Arg Glu Gly Val
        35                  40                  45
Ser Cys Ile Thr Ser Ser Gly Lys Asp Thr Gln Tyr Leu Asp Ser Val
    50                  55                  60
Lys Gly Arg Phe Thr Ile Ser Arg Asp Asn Thr Lys Asn Thr Val Tyr
65                  70                  75                  80
Leu Gln Met Asn Asn Leu Lys Pro Glu Asp Thr Ala Val Tyr Tyr Cys
                85                  90                  95
Ala Ala Pro Gly Arg Val Arg Gly Ser Cys Asn Gly Arg Trp Asp Tyr
            100                 105                 110
Trp Gly Gln Gly Thr Gln Val Thr Val Ser Ser
        115                 120

<210> 126
<211> 122
<212> PRT
<213> Homo sapiens

<220>
<223> Nb 3178

<400> 126
Gln Val Gln Leu Gln Glu Ser Gly Gly Gly Leu Val Gln Pro Gly Gly
1               5                   10                  15
Ser Leu Arg Leu Ser Cys Ala Ala Ser Gly Phe Thr Leu Asp Tyr Tyr
            20                  25                  30
Pro Ile Gly Trp Phe Arg Gln Ala Pro Gly Lys Glu Arg Glu Gly Val
        35                  40                  45
Ser Cys Ile Ser Ser Ser Asp Gly Ser Thr Arg Tyr Ala Asp Ser Val
    50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Ala Gly Pro Ser Gly Tyr Gly Cys Asn Thr Arg Trp Asp Tyr Trp
            100                 105                 110
Gly Gln Gly Thr Gln Val Thr Val Ser Ser
        115                 120

<210> 127
<211> 126
<212> PRT
<213> Homo sapiens

<220>
<223> Nb 3147

<400> 127
Gln Val Gln Leu Gln Glu Ser Gly Gly Gly Leu Val Gln Ala Gly Gly
1               5                   10                  15
Ser Leu Arg Leu Ser Cys Ala Thr Ser Gly Arg Thr Phe Gly Gly Tyr
            20                  25                  30
Asn Arg Gly Trp Phe Arg Gln Thr Gln Gly Lys Glu Arg Glu Phe Val
        35                  40                  45
Ala Ala Ile Thr Gly Ser Gly Asp Ile Thr Tyr Ala Asp Ser Val Lys
    50                  55                  60
Gly Arg Phe Thr Ile Gly Arg Asp Asn Thr Lys Asn Met Val Phe Leu
65                  70                  75                  80
Gln Met Asp Ser Leu Glu Pro Glu Asp Thr Ala Val Tyr Leu Cys Ala
                85                  90                  95
Ser Ser Ser Arg Phe Ser Gly Thr Leu Trp Ala Gly Gln Thr Pro Asn
            100                 105                 110
Tyr Asp Tyr Trp Gly Gln Gly Thr Gln Val Thr Val Ser Ser
        115                 120                 125

<210> 128
<211> 121
<212> PRT
<213> Homo sapiens

<220>
<223> Nb 3196

<400> 128
Gln Val Gln Leu Gln Glu Ser Gly Gly Gly Leu Val Gln Pro Gly Gly
1               5                   10                  15
Ser Leu Arg Leu Ser Cys Ala Ala Ser Gly Leu Ser Leu Asp Tyr Tyr
            20                  25                  30
Ala Ile Gly Trp Phe Arg Gln Ala Pro Gly Lys Glu Arg Glu Gly Val
        35                  40                  45
Ser Cys Ile Ser Ser Ser Asp Gly Ser Thr Arg Tyr Ala Asp Ser Val
    50                  55                  60
Lys Gly Arg Phe Thr Ile Ser Arg Asp Asn Ala Lys Asn Thr Val Tyr
65                  70                  75                  80
Leu Gln Met Asn Ser Leu Lys Pro Glu Asp Thr Ala Val Tyr Tyr Cys
                85                  90                  95
Ala Thr Asp Pro Tyr Asn Ser Cys Thr Gly Pro Tyr Asn Tyr Trp Gly
            100                 105                 110
Gln Gly Thr Gln Val Thr Val Ser Ser
        115                 120

<210> 129
<211> 119
<212> PRT
<213> Homo sapiens

<220>
<223> VHH3132 - Fig.1

<400> 129
Gln Val Gln Leu Gln Glu Ser Gly Gly Gly Leu Val Gln Ala Gly Gly
1               5                   10                  15
Ser Leu Arg Leu Ser Cys Ala Ala Ser Arg Met Met Ser Ser Leu Ser
            20                  25                  30
Ala Met Gly Trp Phe Arg Gln Ala Pro Gly Lys Gln Arg Glu Leu Val
        35                  40                  45
Ala Thr Ile Thr Arg Asp Gly Thr Pro Ile Tyr Ala Asp Ser Val Lys
    50                  55                  60
Gly Arg Phe Thr Ile Ser Arg Asp Asn Thr Lys Asn Thr Val Tyr Leu
65                  70                  75                  80
Gln Met Asn Ser Leu Ile Ser Glu Asp Thr Ala Val Tyr Tyr Cys Thr
                85                  90                  95
Ala Val Asp Thr Met Gly Ala Tyr Trp Gly Gln Gly Thr Gln Val Thr
            100                 105                 110
Val Ser Ser His His His His His His
        115                 120

## Description

### FIELD OF THE INVENTION

The present invention is related to field of immune checkpoints in onco-immunology and their identification as potential targets for the treatment of cancer patients. Besides being considered as therapy targets, early detection of these immune checkpoints could equally be used in diagnosis, and monitoring of patients. Within said field, the present invention provides VHHs to detect and even quantify in a non-invasive way via molecular imaging the human immune checkpoint LAG-3, in particular the expression by tumor-infiltrating leukocytes and demonstrates how said detection is predictive in a response to combination therapy. Moreover, the present invention provides VHHs that have therapeutic ability.

### BACKGROUND TO THE INVENTION

Immune cells are controlled by a plethora of molecules that act as "security brakes" at multiple stages of the immune response. These regulations are important to prevent the destruction of tissues caused by inappropriate and/or disproportionate responses to invading pathogens. Cancer cells exploit such inhibitory immune checkpoints (ICPs) to escape destructive tumor-specific immune responses, which is unfavorable for the patients' outcome.

In 2018, cancer caused 9.6 million deaths and was diagnosed in 18.1 million patients worldwide (*1*). Cancer treatments focusing on reinvigorating exhausted tumor-specific immune cells significantly improved the survival of patients. In particular, the anti-tumor effects observed after monoclonal antibody (mAb)-mediated blockade of the ICPs; cytotoxic T lymphocyte associated protein-4 (CTLA-4, CD152), programmed death-1 (PD-1, CD279) and its ligand PD-L1 (CD274, B7-H1), revolutionized the field of immune-oncology. Therapeutic success was achieved in a fraction of patients and provided clinical evidence that corroborated the preclinical finding that ICPs exert inhibitory effects on immune cells that try to eradicate cancer cells. However, response frequencies to these widely-used ICP-blocking mAbs are suboptimal as a consequence of, amongst others, tumor resistance, absence of tumor-infiltrating lymphocytes (TILs) and the presence of inhibitory myeloid cells (2). Moreover, the occurrence of immune-related adverse events (irAEs) has been a reason to discontinue the use of ICP-blocking mAbs. Taken together, researchers are continuously untangling the biology of inhibitory receptors to increase response rates and to prevent irAEs.

The list of inhibitory ICPs that negatively regulate anti-tumor immune responses is growing. Among these next-generation ICPs, lymphocyte activating gene-3 (LAG-3, CD233) has emerged as an eminent target in the development of cancer treatment and holds substantial prognostic value. With as many as 15 different compounds targeting LAG-3 under (pre)clinical evaluation, it is the most widely studied ICP next to CTLA-4 and PD-1/PD-L1.

LAG-3, also known as CD223, is expressed on numerous immune cell types, where its exact mechanism of action is yet to be fully discovered. However, it is certain that LAG-3 shows a remarkable synergy with PD-1 in promoting immune escape of cancer cells (*3*,*4*). Notably, simultaneous blockade of LAG-3 and PD-1 has shown striking clinical results in melanoma patients that were not responding well to initial PD-1 or PD-L1 monotherapy (*5*,*6*).

The growing list of inhibitory ICPs brings forth the challenge of defining which patients are likely to benefit from ICP-therapy and which inhibitory ICP(s) prevail(s) in the patients' tumors, and therefore should be targeted. Presently, patients are selected for treatment with ICP-blocking mAbs, depending on the level of inhibitory ICP-expression in the tumor microenvironment (TME), determined using immunohistochemistry (IHC). The static picture created by IHC of a tumor biopsy, does not take in account the heterogeneously distributed expression of ICPs. The latter can explain the fact that "non-expressors" do respond to ICP-blocking therapies (7). Contrarily, some patients with IHC samples that show clear expression of ICPs in the TME, are observed not to react to ICP-blocking mAbs (*8*,*9*). This can be due to amongst others, the compensatory expression of other ICPs (*7*,*10*,*11*). Recent studies explored the field of molecular imaging as a more suited alternative for non-invasive detection of ICPs in cancer patients. Molecular imaging allows whole body visualization of prognostic or predictive markers. It can be performed non-invasively and repetitively, which allows quantifying and tracking of the dynamic evolution of expression patterns.

VHHs are the smallest antigen-binding fragment naturally found derived from camelid heavy chain-only antibodies, and generally known as nanobody. Moreover, they hold promise as excellent tools to target proteins in the tumor microenvironment (*7*,*12*). VHHs are small in size, easy to engineer and produce in prokaryotic or yeast cells. Therefore, VHHs targeting LAG-3 could be interesting therapeutic tools, in addition to their diagnostic potential as imaging tracer.

### SUMMARY OF THE INVENTION

Recent advances in the field of onco-immunology led to the discovery of next-generation immune checkpoints. LAG-3, being the most widely studied amongst these next-generation immune checkpoints, is being explored as a target for the treatment of cancer patients. Several antagonistic anti-LAG-3 monoclonal antibodies are being developed and are prime candidates for clinical application. Furthermore, validated therapies targeting CTLA-4, PD-1 or PD-L1 showed that only subsets of patients respond. This finding highlights the need for better tools for patient selection and monitoring. The potential of molecular imaging to detect immune checkpoints noninvasively in cancer is supported by several (pre)clinical studies. Here, we report on a series of VHHs, further characterized hereinafter, to evaluate whole-body mouse LAG-3 (moLAG-3) expression, validated in various syngeneic mouse cancer models using nuclear imaging. The radiolabeled VHHs detected moLAG-3 expression in tumor-infiltrating immune-cells as soon as 1 hour after injection with the highest signal observed in the MC38 colon carcinoma model. The VHH tracer visualized a compensatory upregulation of moLAG-3 on tumor-infiltrating lymphocytes in MC38 tumors of mice treated with moPD-1 blocking antibodies. When moPD-1 blockade was combined with moLAG-3 blockade, a synergic effect on tumor growth delay was observed. Besides the previously reported moLAG-3 VHH 3132, we herewith provide a further set of VHHs binding to human LAG-3, referred to as huLAG-3, with low nanomolar affinity. We developed a series of VHHs, specific for huLAG-3, with good capacity to visualize huLAG-3-expressing tumors in mice. Furthermore, the therapeutic efficacy of huLAG-3 specific VHHs was shown in an experimental setting. In conclusion, these findings consolidate LAG-3 as a next-generation immune checkpoint and support the use of VHHs as tools to therapeutically block and noninvasively monitor the dynamic evolution of LAG-3 expression by tumor-infiltrating leukocytes.

### BRIEF DESCRIPTION OF THE DRAWINGS

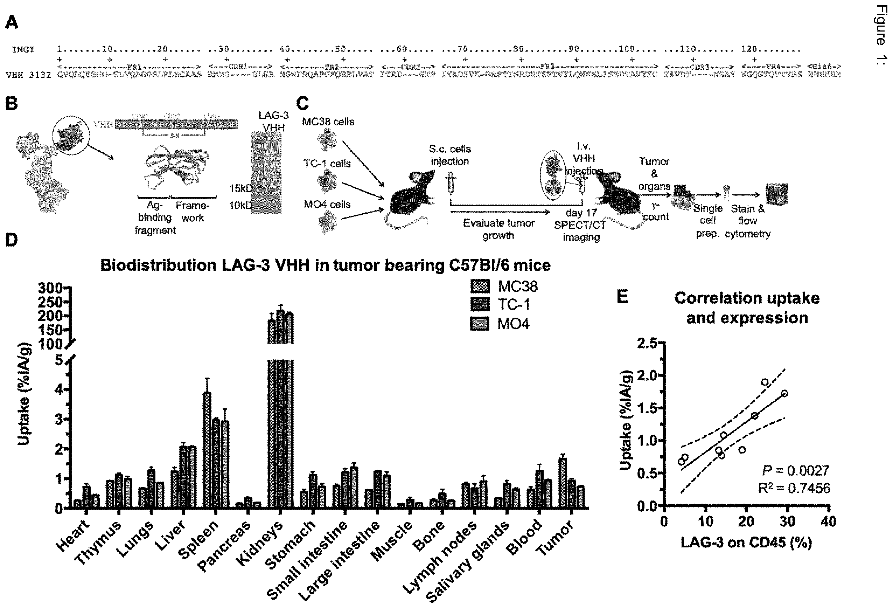

**Figure 1****:** Biodistribution of radiolabeled, moLAG-3 specific VHH 3132 in syngeneic mouse tumor models. (**a**) The amino acid sequence of moLAG-3 specific VHH. (**b**) Structural model of a llama light-chain-deficient antibody from which the VHH is derived and a Coomassie blue-stained SDS-PAGE loaded with 20µg of purified LAG-3 VHH. (**c**) Timeline of the experiment. (**d**) *Ex vivo* γ-counting of isolated organs from MC38 (*n* = 3), TC-1 (*n* = 3) or MO4 (*n* = 3) tumor bearing mice 80 min after injection of radiolabeled LAG-3 VHH, expressed as percentage of injected activity (%IA/g). (**e**) Correlation analysis of tumor accumulation of radiolabeled LAG-3 VHH (in %IA/g) and the percentage of LAG-3⁺ on CD45⁺ cells. The Spearman correlation coefficient R and the corresponding p-value are shown.

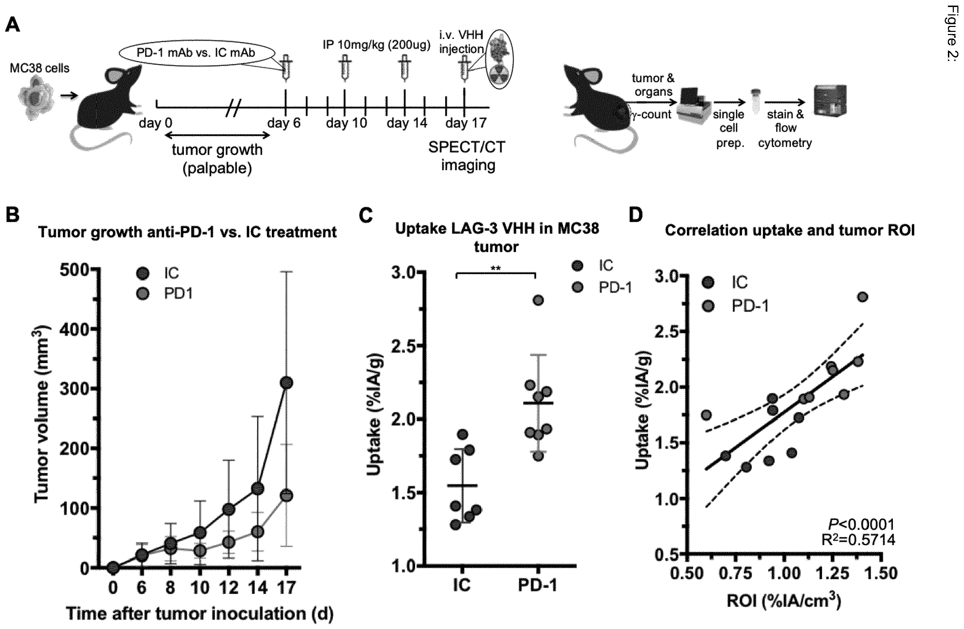

**Figure 2****:** Effect of moPD-1 blockade versus isotype control in MC38 tumor-bearing C57Bl/6 mice on moLAG-3 detection using [^{99m}Tc]-VHH 3132 SPECT/CT imaging. (**a**) Timeline of experiment. (**b**) Growth kinetics of MC38-tumors treated with IC-mAbs (n=7) or PD-1-blocking mAbs (n=8). (**c**) Uptake levels of LAG-3 VHH tracer in MC38-tumors of anti-PD-1 or IC-treated mice, as determined by ex vivo γ-counting. (**d**) Correlation plot and representative axial scans showing computational calculated tumor uptake (in %IA/cm³, x-axis) and the *ex vivo* γ-counting of tumors from IC and PD-1 treated mice (in %IA/g, y-axis).

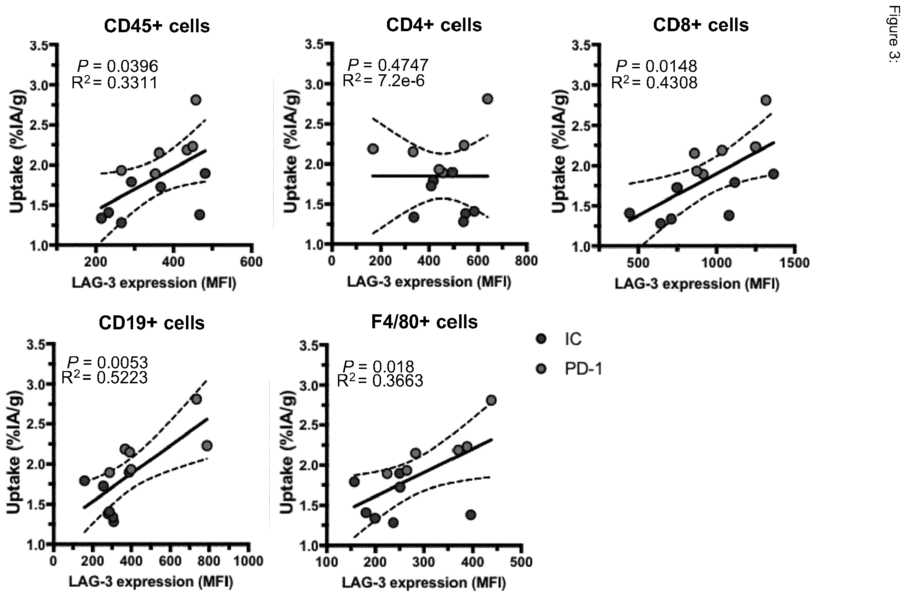

**Figure 3****:** *Ex vivo* analysis of moLAG-3 expression on immune cell populations within the MC38 tumor of anti-PD-1 or IC treated C57BL/6 mice. Correlation plot of moLAG-3 expression (MFI) on different immune cell subsets in MC38 tumors of anti-PD-1 or IC treated mice as analyzed by flow cytometry (x-axis) and the *ex vivo* γ-counting of the tumors in %IA/g (y-axis) after administation of radiolabeled VHH 3132 tracer.

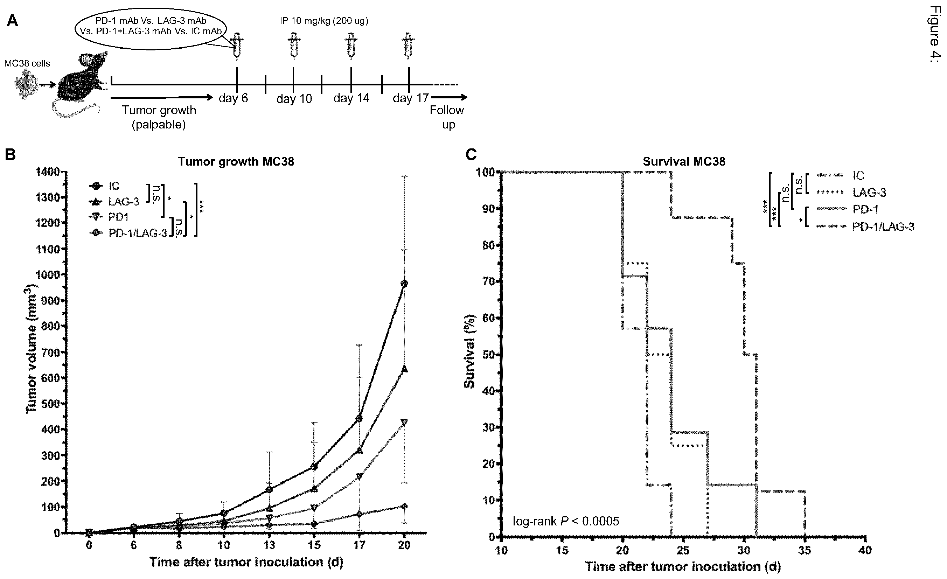

**Figure 4****:** Response of established MC38 tumor-bearing mice to moPD-1 or moLAG-3 monotherapy, combination therapy or IC. (**a**) Timeline of experiment. (**b**) Growth kinetics of MC38 s.c. tumors treated with mAbs targeting moPD-1 (n = 8), moLAG-3 (n = 8), the combination of moPD-1 and moLAG-3 (n = 8) or the corresponding isotype matched control antibodies (n = 8). (c) Kaplan-Meyer curves of tumor-response over time.

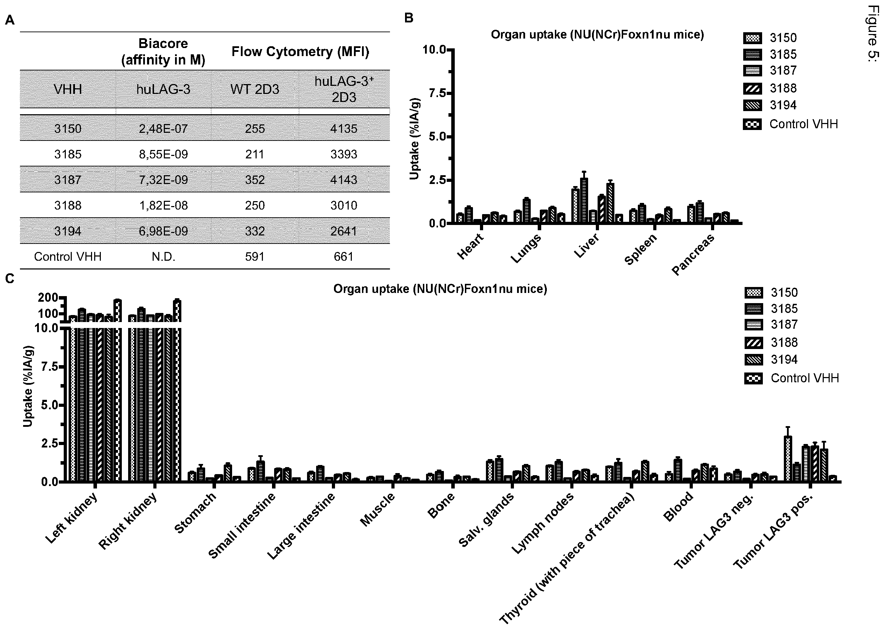

**Figure 5****:** Injection of radiolabeled, huLAG-3 specific, VHHs in tumor bearing mice. (**A**) Characterization of a set of huLAG-3 binding VHHs. (**B-C**) *Ex vivo* γ-counting of isolated organs from NU(NCr)Foxn1nu nude mice 80 min after injection of radiolabeled huLAG-3 VHHs, expressed as percentage of injected activity (%IA/g) . t

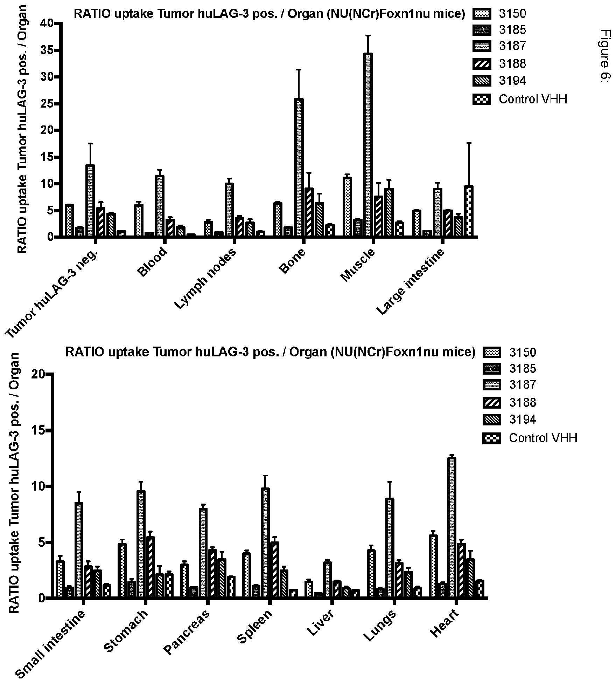

**Figure 6*****:*** Calculated ratios of huLAG-3 specific VHH uptake between tumors, expressing huLAG-3 on their surface, and all the isolated organs from NU(NCr)Foxn1nu nude mice 80 min after injection of radiolabeled huLAG-3 VHHs.

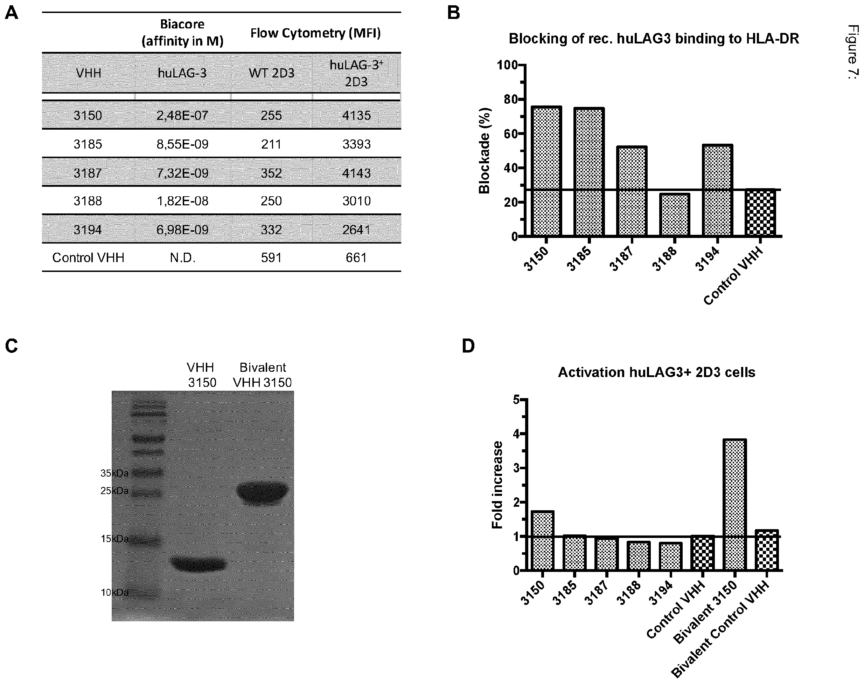

**Figure 7****:** *In vitro* therapeutic efficacy of huLAG-3 binding VHHs. (**A**) Characterization of a set of huLAG-3 binding VHHs. (**B**) Efficiency of a set of huLAG-3 VHHs to block the interaction of recombinant huLAG-3 protein to its ligand MHC-II (HLA-DR). (**C**) Coomassie blue-stained SDS-PAGE loaded with 20µg of purified VHH 3150 in monovalent form (left lane) and bivalent form (right lane). (**D**) Efficiency of VHHs and VHH 3150 in monovalent or bivalent format, to activate huLAG-3 expressing T cells after antigen presentation.

### DETAILED DESCRIPTION OF THE INVENTION

The use of mAbs targeting immune checkpoints like CTLA-4 and PD-1/PD-L1 have emerged in the field of onco-immunology due to their potent effects in a diversity of human cancers. However, there is still a fraction of treated patients that do not respond to this therapy. Additional immune checkpoints, such as LAG-3, have been discovered, offering the potential to overcome resistance in some of these patients, by blocking LAG-3. Its important role in the development of cancer has been addressed in numerous pre-clinical works (*3*,*4*,*13*―*17*). It has been shown that LAG-3 is expressed on T cells, B cells, plasmacytoid dendritic cells, NK cells and macrophages (*13*,*18*―*20*)*.* Its induction is related to the dysfunction of cancer-specific T cells often associated with PD-1 co-expression (*15*,*17*). The latter could be an explanation of therapeutic resistance to single-agent checkpoint blockade in patients. Subsequently, efforts have been made to develop anti-LAG-3 antibodies and explore their anti-cancer efficacy when used as monotherapy or in combination with anti-PD-1 treatment.

It is important to address effective ways to evaluate the expression of immune checkpoints within the tumor in order to select patients and predict their therapy outcome. As mentioned before, immunohistochemistry is proven ineffective in some cases and makes way for improvements (*7*,*12*). In contrast, molecular imaging using small binding moieties like VHHs, has proven to be a method that quickly detects biomarkers with high contrast in a noninvasive way (*7*,*21*). Moreover, it can be performed frequently in the same patient regardless of the location of the tumor. Several clinical evaluations led to the observation that radiolabeled VHHs are ideal candidates to detect tumor biomarkers via imaging with high contrast as soon as 60 min after administration to the patient (12,22-24).

We previously reported a first study characterizing different VHHs targeting the immune checkpoint LAG-3 (*13*). These findings led to the discovery of a compound i.e. VHH 3132 that proved the possibility of whole-body visualization of mouse LAG-3. In this study, we evaluated the use of mouse LAG-3-specific VHH 3132 and a further set of huLAG-3 specific VHHs, to detect LAG-3 expressing tumor-infiltrating immune cells. Therefore, we performed SPECT/CT imaging on tumor mouse models like melanoma (MO4), lung epithelial cancer (TC-1) and colon adenocarcinoma (MC38) inoculated in C57Bl/6 mice using [99mTc] labeled anti-LAG-3 VHHs. Although we acknowledge that subcutaneous injected tumor cells do not represent the same profile of expression and infiltrated cells as orthotropic models, we were mainly interested to see if a diversity of LAG-3 expression between the different tumor models existed and if in that case this diversity could be distinguished when using VHH-mediated noninvasive imaging. Next to the similar signals detected in organs like the thymus, spleen, lymph nodes, etc. (all containing LAG-3 expressing immune cells), we observed differences in the anti-LAG-3 VHH uptake between the different tumor types. Moreover, these uptake levels could be correlated to the ex vivo evaluated LAG-3 expression on CD45+ tumor-infiltrating immune cells using flow cytometry. SPECT/CT images of these scans show the potency of the anti-LAG-3 VHH to map LAG-3 presence in cancer.

As further detailed hereinafter, notwithstanding the fact some of the huLAG-3 specific VHHs according to the invention could be considered to have a low affinity, they were found to have the excellent binding characteristics when analysed using flow cytometry. This did result in a low background uptake and a strong, i.e. high tumor-to-background ratio, making the huLAG-3 VHHs according to the invention particularly useful in whole-body visualisation of LAG-3 via imaging. For example, the huLAG-3 specific VHH 3150 (*infra)* has a low affinity according to biacore, but has the best binding characteristics when analyzed using flow cytometry on huLAG-3+ cells, and is able to block interaction of recombinant huLAG-3 protein to its ligand to similar levels as VHH 3185 which has a high affinity when analyzed using biacore. The huLAG-3 specific VHH 3187 was found to have the highest tumor-to-background ratio, and accordingly represents one of the preferred huLAG-3 VHHs in the VHH-mediated noninvasive imaging application of the huLAG-3 VHHs according to the invention .

As described above, LAG-3 expression can act as a compensatory mechanism that leads to therapeutic resistance of PD-1 immune checkpoint blockade in cancer patients (6,15,17). We believe to be the first to explore the effects of PD-1 treatment on the expression and distribution of LAG-3 using molecular imaging. For this experiment, MC38 tumor bearing mice were treated with PD-1 blocking antibodies administered intraperitoneally 3 times at 10 mg/kg. We observed a significant decrease in tumor growth compared to isotype control treatment as a result of PD-1 blockade. Moreover, noninvasive imaging of these mice using anti-LAG-3 VHH revealed a significantly higher uptake in lymph nodes and their tumor relative to the IC group. Following imaging, LAG-3 expression in the tumor and spleen was evaluated using flow cytometry. PD-1 treated mice revealed a significantly higher infiltration of CD8+ T cells in their tumor as well as a significantly higher cumulative percentage of LAG-3 on CD45+ cells compared to the IC group. Moreover, a positive correlation was found between uptake levels of anti-LAG-3 VHH in the tumor and the mean fluorescence intensity levels of LAG-3 expression on CD8+ T cells, CD19+ B cells and F4/80+ macrophages. These findings support the idea of compensatory upregulation of immune checkpoint LAG-3 in the tumor after the blockade of, in this case, PD-1. Moreover, the enhanced uptake of radiolabeled anti-LAG-3 VHH in PD-1 treated tumors was visible on SPECT/CT images with high contrast as fast as 1h after injection, which proves its capacity to quickly detect the diverse LAG-3 presence in a noninvasive and quantitative manner.

Next, we evaluated the anti-cancer effects of LAG-3 blockade combined with anti-PD-1 treatment in MC38 tumors as we observed a compensatory upregulation of LAG-3 after single-agent PD-1 blockade. Mice injected with only LAG-3 blocking mAbs did not show a decrease in tumor growth compared to the IC. However, when combined with PD-1 blockade, its anti-cancer effect surpasses the marginal, yet significant, decrease in tumor growth when using PD-1 as a monotherapy. Therefore, we can conclude a synergic effect of blocking PD-1 as well as its compensatory upregulated checkpoint partner LAG-3 in the MC38 tumor model. Moreover, evaluation of the spleen of the treated mice showed a significant increase in size when LAG-3 alone or combined with PD-1 was blocked. This is a phenomenon that has previously been observed in LAG-3-deficient mice (25). This could be interpreted as evidence for immunological activity directed against the tumor as previously described by Vignali et al (26). However, LAG-3 blockade alone was not enough to control tumor growth emphasizing the need to combine treatment regimens in regard to immune checkpoint blockade.

Taken together, these results warranted further translation of VHHs capable of imaging LAG-3 to the clinic, as we were able to prove that they are ideal candidates to map the dynamic expression of LAG-3 in cancer in a noninvasive way. The recent developments of several mAbs targeting LAG-3 for the treatment of cancer patients warranted further research in the development of diagnostic tools to evaluate LAG-3 expression in humans, and did result in the identification of following huLAG-3 specific VHHs. Moreover, as VHHs hold potential therapeutic value, we evaluated a set of huLAG-3 specific VHHs as anti-cancer drugs. We were able to show that some of these huLAG-3 VHHs were able to block the interaction of huLAG-3 with the canonical ligand HLA-DR. Furthermore, and in particular VHH 3150 was able to reinvigorate the activity of huLAG-3 expressing T cells. The effect of VHH 3150 was enhanced by changing its monovalent format to a bivalent one.

As used herein, the terms "single domain antibody (VHH)" and "nanobody" have the same meaning referring to a variable region of a heavy chain of a cloned antibody, and construct a single domain antibody (VHH) consisting of only one heavy chain variable region. It is the smallest antigen-binding fragment with complete function.

As used within the context of the present invention the huLAG-3 VHHs, also referred to as huLAG-3 VHHs consist of 4 framework regions (FR1 to FR4 respectively) and 3 complementarity determining regions (CDR1 to CDR3 respectively), in which at least one of the CDR1, CDR2 and CDR3 sequence is chosen from the group consisting of the CDR1, CDR2, CDR3 sequences respectively listed in Table 1(a-c); or from the group of CDR1, CDR2 and CDR3 sequences, respectively, that have at least 80%, preferably at least 90%, more preferably at least 95%, even more preferably at least 99% "sequence identity" (as defined herein) with at least one of the CDR1, CDR2 and CDR3 sequences, respectively, listed in Table 1(a-c); and/or in which the 4 framework regions (FR1 to FR4 respectively) is chosen from the group consisting of the FR1, FR2, FR3, and FR4 sequences respectively listed in Table 2(a-d); or from the group of FR1, FR2, FR3, and FR4 sequences, respectively, that have at least 80%, preferably at least 90%, more preferably at least 95%, even more preferably at least 99% "sequence identity" (as defined herein) with at least one of the FR1, FR2, FR3, and FR4 sequences, respectively, listed in Table 2(a-d).

**Table 1a - CDR1 sequences of huLAG-3 VHHs according to the invention**

| VHH ID | CDR1 | (SEQ ID) |
|---|---|---|
| 3185 | GFTFDDYA | 1 |
| 3188 | GFTFDDYA | 2 |
| 3176 | GFTFDDYA | 3 |
| 3150 | GFTFDDYA | 4 |
| 3187 | GITFDDYA | 5 |
| 3148 | GFIFDDYA | 6 |
| 3194 | GFTLDYYA | 7 |
| 3195 | GFTLDYYA | 8 |
| 3178 | GFTLDYYP | 9 |
| 3183 | GFTLDGYA | 10 |
| 3197 | GFTLDHAA | 11 |
| 3189 | GFTWDYSA | 12 |
| 3180 | GFSVDNYA | 13 |
| 3196 | GLSLDYYA | 14 |
| 3147 | GRTFGGYN | 15 |
| 3202 | GTIFSINV * | 16 |

**Table 1b - CDR2 sequences of huLAG-3 VHHs according to the invention**

| VHH ID | CDR2 | (SEQ ID) |
|---|---|---|
| 3195 | ISSSDGST | 17 |
| 3196 | ISSSDGST | 18 |
| 3194 | ISSSDGST | 19 |
| 3185 | ISSSDGST | 20 |
| 3180 | ISSSDGST | 21 |
| 3178 | ISSSDGST | 22 |
| 3150 | ISSSDGST | 23 |
| 3188 | IRSSDGST | 24 |
| 3197 | IRSSDGST | 25 |
| 3187 | IRSSDDST | 26 |
| 3176 | MRSSDGST | 27 |
| 3148 | IRSSDGNT | 28 |
| 3183 | ISSSGGTT | 29 |
| 3147 | ITGSG-DI | 30 |
| 3189 | ITSSGKDT | 31 |
| 3202 | ITSRGSA- | 32 |

**Table 1c ― CDR3 sequences of huLAG-3 VHHs according to the invention**

| VHH ID | CDR3 | (SEQ ID) |
|---|---|---|
| 3194 | ------ATDRYGTCTGPYDN | 33 |
| 3195 | ------ATDHYGDCKGPYDN | 34 |
| 3176 | ------ASDPWRECKGPYDS | 35 |
| 3180 | ------ARDPFQTCTGPYDS | 36 |
| 3187 | ------ARDVYSDCKGPYDS | 37 |
| 3148 | ------ATDSYSDCKGPYDY | 38 |
| 3183 | ------AADSYGDCKGPYDY | 39 |
| 3150 | ------AADRYRDCRGPYDY | 40 |
| 3188 | ------AADRYRDCRGPYDY | 41 |
| 3197 | ------ATDKFADCKGPYDY | 42 |
| 3196 | ------ATDPYNSCTGPYNY | 43 |
| 3178 | -----AAGPSGYGCNTRWDY | 44 |
| 3189 | ----AAPGRVRGSCNGRWDY | 45 |
| 3185 | ------AAREGDSCSGRWDY | 46 |
| 3202 | ------NALAVQTADGVSDY | 47 |
| 3147 | ASSSRFSGTLWAGQTPNYDY | 48 |

**Table 2a - FR1 sequences of huLAG-3 VHHs according to the invention**

| VHH ID | FR1 | (SEQ ID) |
|---|---|---|
| 3188 | QVQLQESGGGLVHPGGSLRLSCAAS | 49 |
| 3189 | QVQLQESGGGLVQPGGSLKLSCAAS | 50 |
| 3194 | QVQLQESGGGLVQPGGSLRLSCAAS | 51 |
| 3183 | QVQLQESGGGLVQPGGSLRLSCEAS | 52 |
| 3180 | QVQLQESGGGLVQPGGSLRLSCAAS | 53 |
| 3196 | QVQLQESGGGLVQPGGSLRLSCAAS | 54 |
| 3178 | QVQLQESGGGLVQPGGSLRLSCAAS | 55 |
| 3195 | QVQLQESGGSLVQPGGSLRLSCAAS | 56 |
| 3197 | QVQLQESGGDSVQPGGSLRLSCAAS | 57 |
| 3147 | QVQLQESGGGLVQAGGSLRLSCATS | 58 |
| 3148 | QVQLQESGGGLVQAGGSLRLSCAAS | 59 |
| 3150 | QVQLVESGGGLVQAGESLRLSCAAS | 60 |
| 3176 | QVQLQESGGGLVQAGGSLRLSCAAS | 61 |
| 3185 | QVQLQESGGGLVQAGGSLRLSCAAS | 62 |
| 3187 | QVQLQESGGGLVQAGGSLRLSCAAS | 63 |
| 3202 | QVQLQESGGGLVQAGGSLRLSCAAS | 64 |
| | **** **** . * : . * ** : *** : * | |

**Table 2b - FR2 sequences of huLAG-3 VHHs according to the invention**

| VHH ID | FR2 | (SEQ ID) |
|---|---|---|
| 3196 | IGWFRQAPGKEREGVSC | 65 |
| 3197 | IGWFRQAPGKEREGVSC | 66 |
| 3195 | IGWFRQAPGKEREGVSC | 67 |
| 3194 | IGWFRQAPGKEREGVSC | 68 |
| 3189 | IGWFRQAPGKEREGVSC | 69 |
| 3188 | IGWFRQAPGKEREGVSC | 70 |
| 3187 | IGWFRQAPGKEREGVSC | 71 |
| 3185 | IGWFRQAPGKEREGVSC | 72 |
| 3178 | IGWFRQAPGKEREGVSC | 73 |
| 3176 | IGWFRQAPGKEREGVSC | 74 |
| 3150 | IGWFRQAPGKEREGVSC | 75 |
| 3148 | IGWFRQAPGKEREGVSC | 76 |
| 3180 | IGWFRQAPGKQREGVSC | 77 |
| 3183 | IGAFRQAPGKEREAVSC | 78 |
| 3147 | RGWFRQTQGKEREFVAA | 79 |
| 3202 | MGWYRQALGKQRELVAL | 80 |
| | * : ** : ** : ** * : | |

**Table 2c - FR3 sequences of huLAG-3 VHHs according to the invention**

| VHH ID | FR3 | (SEQ ID) |
|---|---|---|
| 3150 | YYADSVKGRFTISSDNAENTVYLQMNSLKPEDTAVYYC | 81 |
| 3188 | YYADSVKGRFTISSDNAENTVYLQMNSLKPEDTAVYYC | 82 |
| 3187 | YYADSVKGRFTISSDNAKNTVYLQMNSLKPEDTAVYYC | 83 |
| 3148 | YYADSVKGRFTISSDNAKNTVYLQMNSLKPEDTAVYYC | 84 |
| 3176 | YYADSVKGRFTISSDNAKNTVYLQMNSLKPEDTAVYYC | 85 |
| 3185 | RYADSVKGRFTISSDNAKNTVRLQMDSLKPEDTAVYYC | 86 |
| 3197 | YYADSVKGRFTISKDNAKNTVYLQMNSLKPEDTAVYYC | 87 |
| 3202 | HLADSVKGRFTISGDNAKNTVYLQMNSLKAEDTAVYYC | 88 |
| 3194 | YYADSVKGRFTISRDNAKNTVYLQMNSLKPEDTAVYYC | 89 |
| 3178 | RYADSVKGRFTISRDNAKNTVYLQMNSLKPEDTAVYYC | 90 |
| 3196 | RYADSVKGRFTISRDNAKNTVYLQMNSLKPEDTAVYYC | 91 |
| 3180 | KYADSVKGRFTISRDNAKNTVYLQMNSLKPEDTAVYYC | 92 |
| 3183 | YYADSVKGRFTISRDNAKNMVYLQMNSLKPEDTAVYYC | 93 |
| 3189 | QYLDSVKGRFTISRDNTKNTVYLQMNNLKPEDTAVYYC | 94 |
| 3195 | YYADSVKGRFAISRDNVKNTVFLQMNSLKPEDTAVYYC | 95 |
| 3147 | TYADSVKGRFTIGRDNTKNMVFLQMDSLEPEDTAVYLC | 96 |
| | ******* : *. **. : * * *** : . * : . ****** * | |

**Table 2d - FR4 sequences of huLAG-3 VHHs according to the invention**

| VHH ID | FR4 | (SEQ ID) |
|---|---|---|
| 3197 | WGQGTQVTVSS | 97 |
| 3202 | WGQGTQVTVSS | 98 |
| 3195 | WGQGTQVTVSS | 99 |
| 3196 | WGQGTQVTVSS | 100 |
| 3194 | WGQGTQVTVSS | 101 |
| 3189 | WGQGTQVTVSS | 102 |
| 3188 | WGQGTQVTVSS | 103 |
| 3185 | WGQGTQVTVSS | 104 |
| 3183 | WGQGTQVTVSS | 105 |
| 3180 | WGQGTQVTVSS | 106 |
| 3178 | WGQGTQVTVSS | 107 |
| 3150 | WGQGTQVTVSS | 108 |
| 3148 | WGQGTQVTVSS | 109 |
| 3147 | WGQGTQVTVSS | 110 |
| 3176 | WGRGTQVTVSS | 111 |
| 3187 | WGRGTQVTVSS | 112 |
| | ** : ******** | |

For the purposes of comparing two or more amino acid sequences, the percentage of ‴sequence identityʺ between a first amino acid sequence and a second amino acid sequence may be calculated by dividing [the number of amino acid residues in the first amino acid sequence that are identical to the amino acid residues at the corresponding positions in the second amino acid sequence] by [the total number of nucleotides in the first amino acid sequence] and multiplying by [100%], in which each deletion, insertion, substitution or addition of an amino acid residue in the second amino acid sequence - compared to the first amino acid sequence - is considered as a difference at a single amino acid residue (position), i.e. as an "amino acid difference" as defined herein. Alternatively, the degree of sequence identity between two amino acid sequences may be calculated using a known computer algorithm, such as those mentioned above for determining the degree of sequence identity for nucleotide sequences, again using standard settings. Usually, for the purpose of determining the percentage of "sequence identity" between two amino acid sequences in accordance with the calculation method outlined hereinabove, the amino acid sequence with the greatest number of amino acid residues will be taken as the "first" amino acid sequence, and the other amino acid sequence will be taken as the "second" amino acid sequence.
Also, in determining the degree of sequence identity between two amino acid sequences, the skilled person may take into account so-called "conservative" amino acid substitutions, which can generally be described as amino acid substitutions in which an amino acid residue is replaced with another amino acid residue of similar chemical structure and which has little or essentially no influence on the function, activity or other biological properties of the polypeptide. Such conservative amino acid substitutions are well known in the art, for example from WO 04/037999, GB-A-2 357 768, WO 98/49185, WO 00/46383 and WO 01/09300; and (preferred) types and/or combinations of such substitutions may be selected on the basis of the pertinent teachings from WO 04/037999 as well as WO 98/49185 and from the further references cited therein.
Such conservative substitutions preferably are substitutions in which one amino acid within the following groups (a) - (e) is substituted by another amino acid residue within the same group: (a) small aliphatic, nonpolar or slightly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic, nonpolar residues: Met, Leu, He, Val and Cys; and (e) aromatic residues: Phe, Tyr and Trp.
Particularly preferred conservative substitutions are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; He into Leu or into Val; Leu into He or into Val; Lys into Arg, into Gln or into Glu; Met into Leu, into Tyr or into He; Phe into Met, into Leu or into Tyr; Ser into Thr; Thr into Ser; Trp into Tyr; Tyr into Trp; and/or Phe into Val, into He or into Leu. Any amino acid substitutions applied to the polypeptides described herein may also be based on the analysis of the frequencies of amino acid variations between homologous proteins of different species developed by Schulz et al., Principles of Protein Structure, Springer- Verlag, 1978, on the analyses of structure forming potentials developed by Chou and Fasman, Biochemistry 13: 211, 1974 and Adv. Enzymol, 47: 45- 149, 1978, and on the analysis of hydrophobicity patterns in proteins developed by Eisenberg et al., Proc. Nad. Acad ScL USA 81 : 140-144, 1984; Kyte & Doolittle; J Molec. Biol. 157: 105-132, 198 1, and Goldman et al, Ann. Rev. Biophys. Chem. 15: 321- 353, 1986, all incorporated herein in their entirety by reference.

In a particular embodiment the huLAG-3 VHHs are selected from the group of VHH sequences listed in Table 2; or from VHH sequences that have at least 80%, preferably at least 90%, more preferably at least 95%, even more preferably at least 99% "sequence identity" (as defined herein) with at least one of the VHH sequences listed in Table 3 below.

In a preferred embodiment the huLAG-3 VHHs are selected from VHH 3150 or VHH 3187; or from VHH sequences that have at least 80%, preferably at least 90%, more preferably at least 95%, even more preferably at least 99% "sequence identity" (as defined herein) with at least one of VHH 3150 or VHH 3187.

The invention in its broadest sense also comprises derivatives of the VHHs of the invention. Such derivatives can generally be obtained by modification, and in particular by chemical and/or biological (e.g enzymatical) modification, of the VHHs of the invention and/or of one or more of the amino acid residues that form the VHHs of the invention. Examples of such modifications, as well as examples of amino acid residues within the VHH sequence that can be modified in such a manner (i.e. either on the protein backbone but preferably on a side chain), methods and techniques that can be used to introduce such modifications and the potential uses and advantages of such modifications will be clear to the skilled person.

For example, such a modification may involve the introduction (e.g. by covalent linking or in another suitable manner) of one or more functional groups, residues or moieties into or onto the VHHs of the invention, and in particular of one or more functional groups, residues or moieties that confer one or more desired properties or functionalities to the VHHs of the invention. Example of such functional groups will be clear to the skilled person.

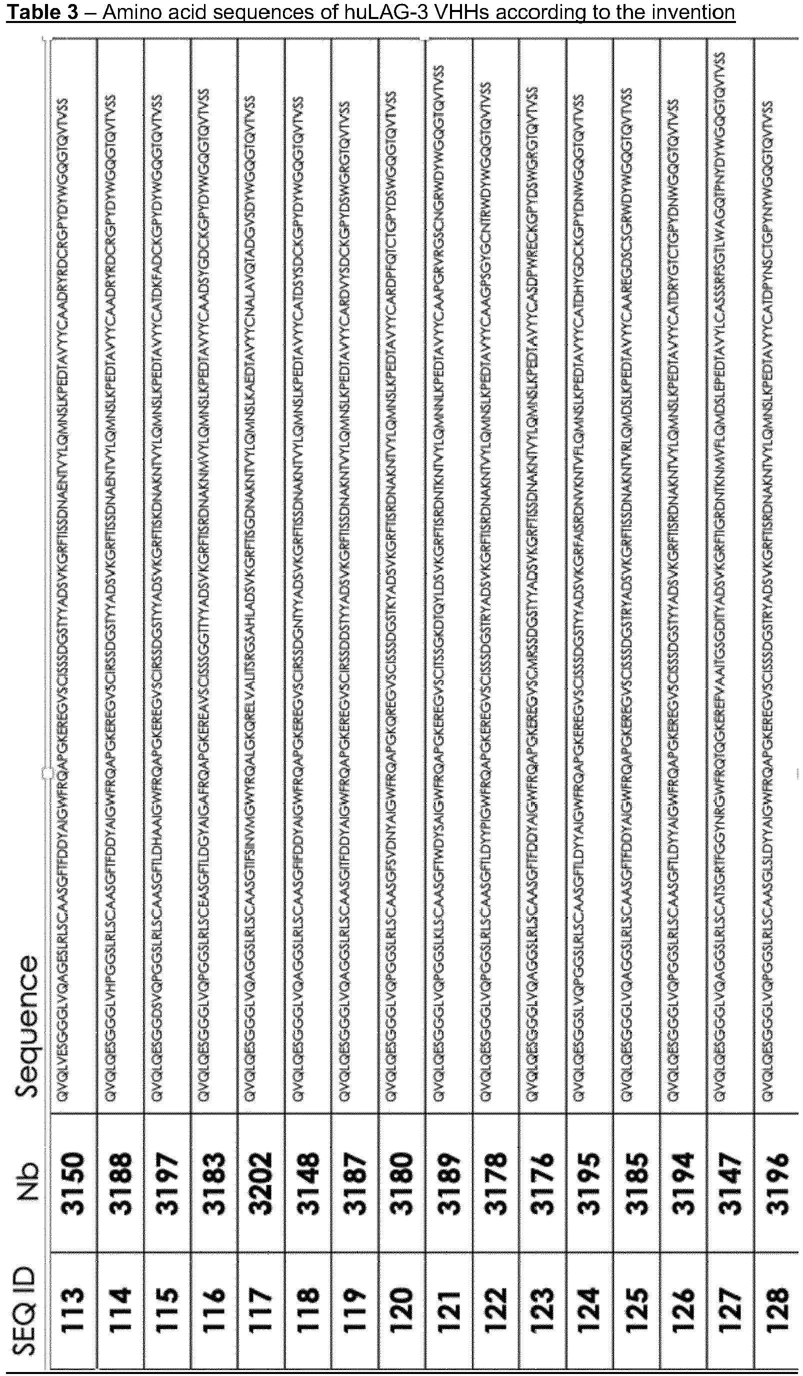

For example, such modification may comprise the introduction of one or more detectable labels or other signal-generating groups or moieties, depending on the intended use of the labelled VHH. Suitable labels and techniques for attaching, using and detecting them will be clear to the skilled person, and for example include, but are not limited to, fluorescent labels (such as fluorescein, IRDye800, IRDye680, FNIR, isothiocyanate, rhodamine, phycoerythrin, phycocyanin, allophycocyanin, o-phthaldehyde, and fluorescamine and fluorescent metals such as Eu or others metals from the lanthanide series), phosphorescent labels, chemiluminescent labels or bioluminescent labels (such as luminal, isoluminol, theromatic acridinium ester, imidazole, acridinium salts, oxalate ester, dioxetane or GFP and its analogs ), radio-isotopes (such as 18F, 124I, 89Zr, 225Ac, 211At, 3H, 125I, 32P, 35S, 14C, 51Cr, 36CI, 57Co, 58Co, 59Fe, and 75Se), metals, metals chelates or metallic cations (for example metallic radio-isotopes such as 99mTc, 123I, 177Lu, 1111n, 131I, 97Ru, 64Cu, 67Cu, 67Ga, and 68Ga or other metals or metallic cations that are particularly suited for use in in vivo, in vitro or in situ diagnosis and imaging, such as (157Gd, 55Mn, 162Dy, 52Cr, and 56Fe), as well as chromophores and enzymes (such as malate dehydrogenase, staphylococcal nuclease, delta- V- steroid isomerase, yeast alcohol dehydrogenase, alpha-glycerophosphate dehydrogenase, triose phosphate isomerase, biotinavidin peroxidase, horseradish peroxidase, alkaline phosphatase, asparaginase, glucose oxidase, β-galactosidase, ribonuclease, urease, catalase, glucose-VI-phosphate dehydrogenase, glucoamylase and acetylcholine esterase). Other suitable labels will be clear to the skilled person, and for example include moieties that can be detected using NMR or ESR spectroscopy.

Such labelled VHHs and polypeptides of the invention may for example be used for in vitro, in vivo or in situ assays (including immunoassays known per se such as ELISA, RIA, EIA and other "sandwich assays", etc.) as well as in vivo diagnostic and imaging purposes, depending on the choice of the specific label.

The inventors have found that the huLAG-3 VHHs of the present invention, when labeled with suitable imaging agents, provide good markers for in vivo imaging. Good specificity without abolishing the high huLAG-3 affinity was obtained with superior tumor localization in vivo. Using the examplary radionucleotide 99mTc as a labeling agent having a short half-life of 6 hours, in combination with the rapidly cleared VHHs of the invention, resulted in low background radiation and consequently in superior imaging results compared to the currently available alternatives with conventional antibodies. Hence in a further aspect the present invention provides a method for the imaging of huLAG-3 targets comprising the step of administering a VHH according to the invention.

Besides the aforementioned labels for imaging purposes, the invention also provides other proteins or fusion expression products having the VHHs of the invention. Specifically, the present invention includes any protein or protein conjugate and fusion expression product (i.e. immunoconjugate and fusion expression product) having a heavy chain containing a variable region, as long as the variable region are identical or at least 90% identical, preferably at least 95% identical to the heavy chain of the VHH of the present invention. As known by those skilled in the art, immunoconjugates and fusion expression products include: conjugates formed by binding drugs, toxins, cytokines, radionuclides, enzymes, and other diagnostic or therapeutic molecules to the VHHs or fragments thereof of the present invention.

For therapeutic purposes, the conjugating moiety of the immunoconjugate of the present invention comprises: toxins, such as small molecular toxins or enzyme active toxins from bacteria, fungi, plant or animal origin, including the fragments and/or variants thereof. Examples of cytotoxic agents include, but are not limited to: Auristatins (such as Auristatin E, Auristatin F, MMAE and MMAF), chlortetracycline, methanesol, ricin, ricin A-chain, cobustatin, dokamicin, dorastatin, Adriamycin, daunorubicin, paclitaxel, cisplatin, cc1065, ethidium bromide, mitomycin, etoposide, tenoposide, vincristine, vinblastine, colchicine, dihydroxyanthrax diketone, actinomycin, diphtheria toxin, Pseudomonas exotoxin (PE) A, PE40, Acacia bean toxin, acacia bean toxin A chain, capsule root toxin A chain, α-sarcissus, gelonin, mitogellin, retstrictocin, phenomycin, enomycin, curicin, croton toxin, calicheamicin, Sapaonaria officinalis inhibitors, as well as glucocorticoids and other chemotherapy agents, and radioisotopes such as At211, I131, I125, Y90, Re186, Re188, Sm153, Bi212 or 213, P32 and Lu, including Lu177. VHHs can also be conjugated to anticancer prodrug activating enzymes that can convert prodrugs into the active forms.

Generally, the VHHs and polypeptides described herein, can be used for all applications known to the skilled artisan in field of VHHs, in particular, the VHHs, and polypeptides described herein can be used in the prevention, diagnosis and/or treatment of diseases and disorders associated with huLAG-3 expression, and in particular with diseases and disorders associated with or characterised by an over-expression of huLAG-3 (i.e. in certain tissues or cells). Examples of such diseases and disorders will be clear to the skilled person, and include various forms of cancers and tumors, such as those mentioned herein, as well as amongst others solid tumors (advanced and/or metatstatic), non-small-cell lung cancer, glioblastoma, melanoma, breast-, hematological-, head and neck-, liver-, gastric-, kidney-, oesophageal- and colon-cancer,

The invention accordingly also provides a composition. Preferably, said composition is a pharmaceutical composition comprising the above VHH or active fragment or fusion protein thereof, and a pharmaceutically acceptable carrier. In general, these materials can be formulated in non-toxic, inert, and pharmaceutically acceptable aqueous carrier media wherein the pH is generally about 5-8, preferably about 6-8, although the pH can be varied with the nature of the formulation material and the condition to be treated. The formulated pharmaceutical compositions can be administered by conventional routes including, but not limited to, intratumoral, intraperitoneal, intravenous, or topical administration.

The pharmaceutical composition of the present invention can be directly used to bind LAG-3 and thus can be used to treat tumors (*supra*)*.* In addition, other therapeutic agents can also be used at the same time.

The pharmaceutical composition of the present invention contains a safe and effective amount (for example, 0.001-99 wt %, preferably 0.01-90 wt %, and more preferably 0.1-80 wt %) of the above-mentioned nanobodies or active fragment or active fusion protein thereof (or their conjugates) and pharmaceutically acceptable carriers or excipients. Such carriers include, but are not limited to: saline, buffer, dextrose, water, glycerol, ethanol, and the combinations thereof. The drug formulation should be suitable for the mode of administration. The pharmaceutical composition of the present invention may be prepared in the form of an injection, for example, by a conventional method using physiological saline or an aqueous solution containing glucose and other adjuvant. Pharmaceutical compositions such as injections and solutions are preferably made under aseptic conditions. The amount of active ingredient, i.e. of nanobodies or active fragment or active fusion protein, administered is a therapeutically effective amount, for example, about 10 micrograms/kilogram body weight to about 50 milligrams/kilogram body weight per day. In addition, the polypeptides of the invention can also be used with other therapeutic agents. For imaging purposes the amount of active ingredient is typically between and about 1 micrograms/kilogram body weight to about 1 milligrams/kilogram body weight per day.

When a pharmaceutical composition is used, a safe and effective amount of the immunoconjugate is administered to the mammal, wherein the safe and effective amount is usually at least about 1 micrograms/kilogram body weight, and in most cases, no more than about 50 mg/kilogram body weight, preferably the dose is about 10 micrograms/kilogram body weight to about 10 milligrams/kilogram body weight. Of course, factors such as the route of administration and the patient's health status should be considered to define the specific doses, all of which are within the skills of skilled physicians.

The Main Advantages of Invention Include:
(a) The VHHs of the present invention targets both advanced and metastatic tumors simultaneously with whole body imaging for the localization, qualitative and quantitative measurement as well as staging of cancer.
(b) The VHHs of the present invention has small size and high specificity and can penetrate the tumor tissue more effectively and is more easily excreted by the body, thereby reducing the radiation dose to the non-targeted tissues.
(c) The VHHs of the present invention have a short half-life and a shorter diagnostic time. and the results can quickly be obtained.
(d) The VHHs of the present invention have high specificity, accurate detection and can avoid local false-negative.
(e) The specific binding of the huLAG-3 VHHs of the present invention to LAG-3 VHHs blocks the interaction of huLAG-3 with the canonical ligand HLA-DR, rendering them particularly useful in anti-cancer therapy and in screening methods to identify further anti-cancer agents, in for example a competitive binding assay.

### EXAMPLES

### Materials and Methods

### 1. Mice, Cell lines & Reagents

Female, 6 to 12-week-old C57BL/6 or NU(NCr)Foxn1nu mice were purchased from Charles River (Ecully, France). Approval of Ethical Committee for laboratory animals of the Vrije Universiteit Brussel was granted prior to execution of the experiments. All animal studies were performed in accordance to the European guidelines for animal experimentation (ethical dossiers 15-214-1 and 19-214-6). The MC38 mouse colon adenocarcinoma cell line, the human embryonal kidney (HEK) 293T cells was obtained from the American Type Culture Collection (ATCC, Molsheim Cedex, France), the MO4 melanoma cell line was kindly provided by Dr. K. Rock (University of Massachusetts Medical center). These cell lines were cultured in Dulbecco's modified Eagle's medium (DMEM) (Sigma-Aldrich, Zwijndrecht, Belgium) supplemented with 10% fetal bovine serum (FBS) (Harlan, Horst, The Netherlands), 2 mmol/l L-glutamine (L-Glu) (Sigma-Aldrich, Zwijndrecht, Belgium), 100 U/ml penicillin and 100 µg/ml streptomycin (PS) (Sigma-Aldrich, Zwijndrecht, Belgium). The TC-1 mouse lung epithelial cell line was kindly provided by T.C. Wu (Johns Hopkins University, Baltimore, Maryland, USA) and cultured in RPMI 1640 medium (Sigma-Aldrich, Zwijndrecht, Belgium), supplemented with 10% fetal clone I (FCI) serum (Harlan, Horst, The Netherlands), L-Glu, PS, 1 mmol/l sodium pyruvate and non-essential amino acids (Sigma-Aldrich, Zwijndrecht, Belgium), 12.5 mM D(+)-glucose, 1mM Geneticin (G418), 5mM HEPES and 50µM β-mercaptoethanol. 2D3 cells were generated as described before (27) and cultured in Iscove's modified Dulbecco's medium (IMDM) (Thermo Scientific) supplemented with 10% FBS, 2mM L-Glutamine, 100U/mL penicillin and 100µg/mL streptomycine. PBMCs obtained from HLA-DR^{POS} donors were generated by the Blood Transfusion Center of the Brussels University Hospital. These cells were collected by leukaphereses (COBE Spectra Apheresis System, Terumo BCT, USA. Recombinant human LAG-3 protein (Fc Chimera) used for competition studies was obtained from R&D (2319-L3) (Bio-Techne, Abingdon, UK). A PerCP-eFluor710- or PE-labeled antibody specific for mouse LAG-3 (Biolegend, clone eBioC9B7W) or human LAG-3 (eBiosciences, clone 3DS223H) was used in flow cytometry to evaluate mouse or human LAG-3 expression on cells respectively. The antibodies used to discriminate immune populations from mouse organ preparations in flow cytometry were: CD45.2-APC-Cy7 (BD Biosciences, clone 104), CD4-AF700 (BD Biosciences, clone RM4-5), CD8a-V450 (BD Biosciences, clone 53-67), CD19-AF647 (BD Biosciences, clone 1D3), F4/80-BB700 (BD Biosciences, clone T452342). The flow cytometry antibody, targeting human IgG1fc-AF488, was used to detect binding of recombinant LAG-3 to its ligand (A-10631) (Thermo Fisher Scientific).

### 2. VHH quality control

Binding characteristics of mouse and human specific LAG-3 VHHs was evaluated using flow cytometry and biacore as previously described (13). Purity of mouse and human LAG-3 specific VHHs was evaluated on a reducing 12% SDS-PAGE followed by Coomassie blue staining. Lipopolysaccharide (LPS) content was evaluated using a limulus amebocyte lysate (LAL)-test (Endosafe^{®} nexgen-PTS^{™}, Charles River, Ecully, France). Results were below detection limit. VHH R3B23, specific for a multiple myeloma paraprotein (28), was used as a negative control throughout this study.

### 3. Inoculation of tumor cells and monitoring of tumor growth

C57BL/6 mice were subcutaneously injected with 3 × 10⁵ MC38 mouse colon adenocarcinoma cells, MO4 melanoma cells or TC-1 mouse lung epithelial cells. NU(NCr)Foxn1nu nude mice were subcutaneously injected with 1 × 10⁶ TC-1 cells, either lentivirally transduced to express human LAG-3 on their membrane or not in right or left flank respectively. Their wellbeing, i.e. body weight, behavior, physical appearance and tumor ulceration was examined daily. Tumor growth was measured every other day using a caliper and the tumor volume was calculated using the formula: (length × width²)/2. All practices were performed under isoflurane anesthesia (5% for induction, 2.5% for maintenance).

### 4. Immune checkpoint blockade in tumor-bearing mice

C57BL/6 mice bearing MC38 tumors were treated with 200 µg (10 mg/kg) of mAbs targeting mouse PD-1 (Ultra-LEAF^{™} anti-mouse PD-1, clone RPM1-14), mouse LAG-3 (Ultra-LEAF^{™} anti-mouse LAG-3, clone C9B7W) or the corresponding isotype matched control antibodies (Ultra-LEAF^{™} Rat IgG1κ, clone RTK2071 and Ultra-LEAF^{™} Rat IgG2aκ, clone RTK2758) via intraperitoneal injection. All mAbs were purchased from Biolegend (London, United Kingdom).

### 5. VHH [^{99m}Tc] labelings, SPECT-CT imaging, image and biodistribution analysis

VHHs were labeled with [^{99m}Tc] as previously described (13). Mice were intravenously injected with 5 µg of [^{99m}Tc]-labeled LAG-3 specific or control VHH. SPECT-CT imaging was performed using a Vector⁺/CT MiLABS scanner (MILabs, Utrecht, The Netherlands). Mice were anaesthetized by intraperitoneal injection with 75 mg/kg ketamine hydrochloride + 1 mg/kg medetomidine (Ketamidor, Richter Pharma AG, Austria). The organ-specific uptake of each radiotracer was measured using a Wizard² *γ*-counter (PerkinElmer, Waltham, MA, USA). The uptake in each organ/tissue was corrected for decay and calculated as the percentage of injected activity per gram (%IA/g). Image analysis was performed using AMIDE (Medical Image Data Examiner software) and HOROS medical imaging viewer (LGPL license at Horosproject.org).

### 6. Single cell preparation of tumor and spleen

Single-cell suspensions of tumors and spleens of C57BI/6 mice were prepared according to the protocols of Miltenyi Biotec. Briefly, tumors were cut in pieces of approximately 3 mm and transferred to gentleMACS C tubes containing 5 ml ice-cold RPMI 1640 supplemented with 1000 U/ml DNAse I and 100 µl collagenase I. The tumors were homogenized at 37°C for 45 min using the gentleMACS^{™} OCTO dissociator (program: cus_37C_mlmpTu2).

### 7. Flow cytometry

The process of staining cell surface markers was previously described (29). Live/death staining of the single cell preparations was performed using ZombieAqua^{™} (BV510, Biolegend) in PBS. Blockade of Fc*γ*II and Fc*γ*III receptors (CD16/32 antibody, clone 93, BioLegend) as well as staining of cell surface makers was performed in PBS containing 0.5% BSA and 0.02% sodium azide. Cells were acquired on a FACSCelesta or LSRFortessa flow cytometer (BD Biosciences). Data were analyzed using FlowJo X^{®} software (Tree star, Inc., Ashland, OR, USA).

### 8. In vitro competition assays

In vitro competition assays were performed by incubating 2 × 10⁵ HLA-DR positive PBMC cells with a mixture of 4ug human recombinant LAG-3 protein (fc chimera) and VHHs at a final concentration of 500nM. After 1 hour incubation at 4°C, cells are washed and presence of recombinant human LAG-3 on cells was detected using an anti-human IgG1fc-AF488 antibody and evaluated using flow cytometry.

### 9. 2D3 assay

HLA-DR positive PBMC cells were pulsed with 50µg/mL gp100₂₈₀₋₂₈₈ peptide and seeded at 2×10⁴ cells per well in a 96-well round bottom plate. 6 × 10⁶ 2D3 cells were transfected with mRNA encoding the TCRα (15µg) and TCRβ (15µg) chain of a TCR recognizing the gp100_{280- 288} peptide in the context of HLA-A2 with or without 2.5µg mRNA encoding LAG-3, as previously described (27). Expression of LAG-3 and the TCR was confirmed by flow cytometry. A total of 2×10⁵ LAG-3^{POS} or LAG-3^{NEG} and TCR^{POS} 2D3 cells were added per well to the PBMC cells. GFP expression by the 2D3 cells served as a measure of TCR signaling was determined in flow cytometry.

### 10. Statistics

All statistical analyses were performed with GraphPad Prism software (version 7.2). All data are represented as mean ± standard deviation (SD). *p*-values were calculated using Kruskal-Wallis tests followed by Dunn's post hoc tests, using Mann-Whitney tests, Spearman correlation tests or log rank tests for survival experiments. The asterisks in the figures indicate statistical significance as follows: **p*<*0.05;* ***p*<*0.01;* ****p*<*0.001;* *****p*<*0.0001; n.s.* not significant. The exact p-value for each experiment is stated in the results section.

### Results

### 1. The accumulation of radiolabeled mouse LAG-3 VHHs positively correlates with the percentage of LAG-3⁺ tumor-infiltrating immune cells in different tumor models

From our previous study, we selected LAG-3 VHH 3132 (Figure 1a, b) to further deliver a proof-of-concept on the applicability of VHHs to quantify LAG-3⁺ immune cells infiltrating into syngeneic tumors via noninvasive imaging (*13*).

In this study we radiolabeled LAG-3 VHH with [^{99m}Tc] and evaluated its biodistribution when injected in C57BL/6 mice bearing MC38, TC-1 or MO4 tumors (Figure 1c). Eighty minutes (min) post-injection of the radiolabeled VHH, we observed accumulation in the kidneys, which is explained by the typical renal accumulation and clearance of VHHs (Figure 1d). We moreover observed accumulation in the thymus, liver, spleen, intestines, lymph nodes and tumors. The uptake of the radiolabeled VHH was the highest in MC38 tumors and lowest in MO4 tumors (Figure 1d, *p = 0.0219*)*.* The uptake of radiolabeled LAG-3 VHH in TC-1 tumors was intermediate and not statistically different from its uptake observed in MC38 or MO4 tumors. Flow cytometry analysis showed that LAG-3 expression was significantly higher on immune cells (CD45⁺) residing in MC38 tumors compared to MO4 tumors (p = *0.0338*), however was not statistically different from that observed on immune cells in TC-1 tumors (p = *0.4081*) (data not shown). This was in line with the observed uptake of radiolabeled LAG-3 VHH, and both tracer tumor uptake levels and percentage of LAG3⁺ tumor-infiltrating cells correlated positively (*R²* = *0.7456*) (*p* = *0.0027*) (Figure 1e).

### 2. Mouse PD-1 blockade causes upregulation of mouse LAG-3 on MC38 tumor-infiltrating immune cells but not in immunological organs like the spleen

In a clinical situation, LAG-3 imaging could be used to identify upregulation of LAG-3 during PD-(L)1 blockage therapy and select patients who could benefit from combination therapy. We therefore investigated if LAG-3 imaging could identify LAG-3 upregulation as a result of PD-1 blockade in a mouse model. C57Bl/6 mice were s.c. injected with MC38 cells after which on day 6, day 10 and day 14 of tumor growth anti-mouse PD-1 or isotype control (IC) mAbs were administered i.p. (Figure 2a). Next, on day 17, mice were i.v. injected with [^{99m}Tc]-labeled LAG-3 VHH, followed by SPECT/CT imaging, *ex vivo* activity biodistribution analysis and flow cytometry analysis of tumor-infiltrating immune cells.
As expected, we observed a slower tumor growth when mice were treated with PD-1 blocking mAbs compared to the IC mAbs (Figure 2b; *p* = *0.0323*) (*14*,*30*). *Ex vivo* activity biodistribution analysis at 80 min post tracer-injection showed a significantly higher uptake in lymph nodes (data not shown, *p* = *0.0037*) and MC38 tumor (Figure 2c, *p* = *0.0022*) as a result of PD-1 treatment. Representative axial and 3D maximum intensity projection (MIP) images show a visible increase of LAG-3 VHH tracer uptake at the tumor site in mice treated with PD-1 blocking mAbs compared to IC mAbs (data not shown). Moreover, we evaluated SPECT/CT images using AMIDE software and calculated activity within the ROI (%IA/cm³), assigned over the tumor mass. These values were correlated to the *γ*-counts obtained through *ex vivo* analysis (Figure 2d) and showed a significant positive correlation (*R²* = *0.5714*) (*p* < *0,0001*).

As we performed flow cytometry analysis on single cell preparations of tumors, we correlated uptake levels of radiolabeled LAG-3 VHH (%IA/g), in the tumors of mice treated with anti-PD-1 or IC antibodies, to the MFI levels of LAG-3 expression on tumor infiltrating immune cells. A positive correlation was for for CD45⁺ cells (*R²* = *0.3311, p* = 0.0396), CD8⁺ T cells (*R²* = *0.4308, p* = *0.0148*), CD19⁺ B cells (*R²* = *0.5223, p* = *0.0053*) and F4/80⁺ macrophages (*R²* = *0.3663, p* = *0.0180*), however not for CD4⁺ T cells (*R²* = *7.2 × 10⁻⁶, p* = *0.4747*) (Figure 3). Moreover, we could observe a significantly higher CD8⁺ T cell infiltration in tumors of PD-1 treated mice relative to IC (data not shown; *p* = *0.0350*)*.* No significant differences in tumor infiltration were shown for CD4⁺ T cells, CD19⁺ B cells or F4/80⁺ macrophages (data not shown). Moreover, we observed an increase in LAG-3 expression on total CD45⁺ immune cells in mice treated with anti-PD-1 mAbs (*p* = *0.0221*) (data not shown). Although not statistically significantly different between PD-1 and IC treated groups, the proportion of LAG-3 expressing CD8⁺ T cells, CD19⁺ B cells and F4/80⁺ macrophages tended to be more pronounced in mice treated with anti-PD-1 mAbs (data not shown). Enhanced expression of LAG-3 on these immune cells infiltrating tumors of mice treated with anti-PD-1 mAbs was further corroborated by an increase in mean fluorescence intensities (MFI), which was statistically different from IC treated group for CD19⁺ B cells (data not shown, *p* = *0.0140*)*.*

### 3. Administration of mouse LAG-3 blocking antibodies enhances antitumor activity of mouse PD-1 inhibitors and increases immunological activity in MC38 tumor model

As we could noninvasively detect the upregulation of LAG-3 on tumor-infiltrating CD45⁺ cells upon PD-1 blockade, we assessed whether the blockade of LAG-3 could have antitumor effects when used alone or act synergistically when combined with anti-PD-1 treatment. Therefore, we s.c. injected C57Bl/6 mice with 3 × 10⁵ MC38 cells after which on day 6, day 10, day 14 and day 17 of tumor growth 200 µg (10 mg/kg) of anti-PD-1 mAbs, anti-LAG-3 mAbs, the combination of both PD-1 and LAG-3 mAbs (10 mg/kg each) or the isotype control mAbs (10 mg/kg each) were i.p. administered (Figure 4a). Treatment of MC38 tumor-bearing mice with LAG-3 or PD-1 monotherapies was only partially efficacious. Statistical difference could be shown when comparing PD-1 treated group to IC group (*p* = *0.0201*). The latter was consistent with the experiment summarized in Figure 3c. As shown in Figure 4b, PD-1/LAG-3 combination therapy was convincingly able to reduce tumor growth relative to IC group (p = *0.0001).* Moreover, PD-1/LAG-3 combination therapy was statistically different from LAG-3 monotherapy (*p* = *0.0201*) but not for PD-1 monotherapy (*p* = *0.5257*)*.* As soon as tumors reached 1500mm³ or ulcerated, mice were sacrificed. Follow-up of the mice revealed a significantly longer survival of only the PD-1/LAG-3 group when compared to IC group (p = *0.0005*) (Figure 4c). Moreover, survival of PD-1/LAG-3 group was statistically different from LAG-3 monotherapy (p = *0.0004*) and PD-1 monotherapy (p = *0.0181*)*.* Spleen weights of mice injected with LAG-3 blocking antibodies as monotherapy were significantly higher relative to IC group (p = *0.0289*) and PD-1 group (p = *0.0211*) (data not shown). Spleen weights of the combination PD-1/LAG-3 group were significantly higher relative to the PD-1 group (p = *0.0474*)*.*

### 4. The In vivo accumulation of radiolabeled human LAG-3 VHHs is specific

As the experiments described above were performed with VHHs that are not cross-reactive to the human form of LAG-3, we needed to develop a separate set of VHHs that are specific for human LAG-3. First, VHHs were evaluated using biacore and flow cytometry to evaluate their specificity and affinity for human LAG-3 (Figure 5a). Secondly, we evaluated the potential of these VHHs to noninvasively detect human LAG-3 in an *in vivo* setting. Therefore, we lentivirally transduced TC-1 lung epithelial tumor cell line to express human LAG-3 protein on their surface and injected these cells subcutaneously in the right flank of NU(NCr)Foxn1nu nude mice. In that way we were able to introduce human LAG-3 expression in a living organism that we are able to study in a preclinical setting. Moreover, to include a negative control, we subcutaneously injected each mice with TC-1 cells not expressing human LAG-3, in the left flank. Next, on day 10 of tumor growth, mice were i.v. injected with [^{99m}Tc]-labeled LAG-3 VHHs, followed by SPECT/CT imaging, *ex vivo* activity biodistribution analysis. The set of evaluated human LAG-3 VHHs did not specifically accumulated in organs of the mice since they are not cross-reactive with mouse LAG-3 (Figure 5b,c). However, we observed specific accumulation of human LAG-3 specific VHHs in the TC-1 tumors expressing human LAG-3 compared to the control VHH (Figure 5c). Moreover, no accumulation could be seen in TC-1 tumors not expressing human LAG-3. Surprisingly, when calculating the ratio of uptake of radiolabeled VHHs in TC-1 tumors expressing LAG-3 (TC-1 hLAG-3 pos.) and other organs, VHH 3187 showed a favorable biodistribution profile as excellent candidate for the noninvasive imaging of human LAG-3 (Figure 6).

### 5. The ability of VHHs to block the interaction of human LAG-3 and its canonical ligand HLA-DR is not predictive for their therapeutic efficacy

Besides evaluating VHHs to noninvasively detect LAG-3, we also examined their use as therapeutic agents. Therefore, we optimized an assay that allows to measure the capacity of VHHs to block the interaction of human LAG-3 and its canonical ligand HLA-DR (MHC-II). Recombinant human LAG-3 (fc chimera) was incubated on A20 cells (4ug), that express HLA-DR, in the presence or not of selected LAG-3 targeting VHHs (500nM) (Figure 7a). After 1 hour incubation on 4°C, the cells were washed and bound LAG-3 was detected using an AF-488 labeled anti-human IgG1 fc antibody for flow cytometry analysis. Nb 3150 and 3185 were able to block the interaction of LAG-3 and its ligand for 75% (Figure 7b). Moreover, considered as moderate blockers are nb 3187 and 3184. No blockade of LAG-3 binding was seen with nb 3188.

In order to evaluate if the VHHs were able to block the inhibitory effects of human LAG-3 on T cells, the following assay was optimized. 2D3 cells, also-known-as Jurkat J76.7 cells, were lentiirally transduced to express human LAG-3. When co-cultured with PBMCs, that acted as antigen-presenting cells, it triggered the T cell receptor on 2D3 cells and induced downstream GFP expression. This is a measure for activation which was evaluated using flow cytometry. However, inhibitory effects of LAG-3 prevent such activation. Moreover, we observed a 2-fold increase of GFP expression when the co-cultures are supplemented with human LAG-3 specific VHH 3150 compared to control VHH (Figure 7d). Moreover, the effect of VHH 3150 could be extended when changing its format from monovalent form to bivalent form (Figure 7c,d).

### References

1. Bray F, Ferlay J, Soerjomataram I, Siegel RL, Torre LA, Jemal A. Global cancer statistics 2018: GLOBOCAN estimates of incidence and mortality worldwide for 36 cancers in 185 countries. CA Cancer J Clin. 2018;68:394-424.
2. Awad RM, De Vlaeminck Y, Maebe J, Goyvaerts C, Breckpot K. Turn Back the TIMe: Targeting Tumor Infiltrating Myeloid Cells to Revert Cancer Progression. Front Immunol. 2018;9:1977.
3. Woo S-R, Turnis ME, Goldberg M V, et al. Immune inhibitory molecules LAG-3 and PD-1 synergistically regulate T-cell function to promote tumoral immune escape. Cancer Res. 2012;72:917-927.
4. Matsuzaki J, Gnjatic S, Mhawech-Fauceglia P, et al. Tumor-infiltrating NY-ESO-1-specific CD8+ T cells are negatively regulated by LAG-3 and PD-1 in human ovarian cancer. Proc Natl Acad Sci U S A. 2010;107:7875-7880.
5. Ascierto PA, Melero I, Bhatia S, et al. Initial efficacy of anti-lymphocyte activation gene-3 (anti-LAG-3; BMS-986016) in combination with nivolumab (nivo) in pts with melanoma (MEL) previously treated with anti-PD-1/PD-L1 therapy. J Clin Oncol. 2017;35:9520.
6. European Society for Medical Oncology. ESMO 2017 Congress Scientific Meeting Report. https://oncologypro.esmo.org/content/download/126251/2385263/file/ESMO-2017-Congress-Scientific-Meeting-Report.pdf.
7. Broos K, Lecocq Q, Raes G, Devoogdt N, Keyaerts M, Breckpot K. Noninvasive imaging of the PD-1:PD-L1 immune checkpoint: Embracing nuclear medicine for the benefit of personalized immunotherapy. Theranostics. 2018;8:3559-3570.
8. Topalian SL, Hodi FS, Brahmer JR, et al. Safety, activity, and immune correlates of anti-PD-1 antibody in cancer. N Engl J Med. 2012;366:2443-2454.
9. Brahmer JR, Drake CG, Wollner I, et al. Phase I study of single-agent anti-programmed death-1 (MDX-1106) in refractory solid tumors: safety, clinical activity, pharmacodynamics, and immunologic correlates. J Clin Oncol. 2010;28:3167-3175.
10. Yarchoan M, Hopkins A, Jaffee EM. Tumor Mutational Burden and Response Rate to PD-1 Inhibition. N Engl J Med. 2017;377:2500-2501.
11. Tang H, Liang Y, Anders RA, et al. PD-L1 on host cells is essential for PD-L1 blockade-mediated tumor regression. J Clin Invest. 2018;128:580-588.
12. Lecocq Q, De Vlaeminck Y, Hanssens H, et al. Theranostics in immuno-oncology using nanobody derivatives. Theranostics. 2019.
13. Lecocq Q, Zeven K, De Vlaeminck Y, et al. Noninvasive Imaging of the Immune Checkpoint LAG-3 Using Nanobodies, from Development to Pre-Clinical Use. Biomolecules. 2019;9.
14. Burova E, Hermann A, Dai J, et al. Preclinical Development of the Anti-LAG-3 Antibody REGN3767: Characterization and Activity in Combination with the Anti-PD-1 Antibody Cemiplimab in Human PD-1xLAG-3-Knockin Mice. Mol Cancer Ther. 2019;18:2051-2062.
15. Huang R-Y, Eppolito C, Lele S, Shrikant P, Matsuzaki J, Odunsi K. LAG3 and PD1 co-inhibitory molecules collaborate to limit CD8+ T cell signaling and dampen antitumor immunity in a murine ovarian cancer model. Oncotarget. 2015;6:27359-27377.
16. Harris-Bookman S, Mathios D, Martin AM, et al. Expression of LAG-3 and efficacy of combination treatment with anti-LAG-3 and anti-PD-1 monoclonal antibodies in glioblastoma. Int J cancer. 2018;143:3201-3208.
17. Huang R-Y, Francois A, McGray AR, Miliotto A, Odunsi K. Compensatory upregulation of PD-1, LAG-3, and CTLA-4 limits the efficacy of single-agent checkpoint blockade in metastatic ovarian cancer. Oncoimmunology. 2017;6:e1249561.
18. Xu J, Chen L-J, Yang S-S, et al. Exploratory trial of a biepitopic CAR T-targeting B cell maturation antigen in relapsed/refractory multiple myeloma. Proc Natl Acad Sci U S A. 2019;116:9543-9551.
19. Triebel F, Jitsukawa S, Baixeras E, et al. LAG-3, a novel lymphocyte activation gene closely related to CD4. J Exp Med. 1990;171:1393-1405.
20. Keane C, Law SC, Gould C, et al. LAG3: a novel immune checkpoint expressed by multiple lymphocyte subsets in diffuse large B-cell lymphoma. Blood Adv. 2020;4:1367-1377.
21. Du Y, Jin Y, Sun W, Fang J, Zheng J, Tian J. Advances in molecular imaging of immune checkpoint targets in malignancies: current and future prospect. Eur Radiol. 2019;29:4294-4302.
22. Keyaerts M, Xavier C, Heemskerk J, et al. Phase I Study of 68Ga-HER2-Nanobody for PET/CT Assessment of HER2 Expression in Breast Carcinoma. J Nucl Med. 2016;57:27-33.
23. Xing Y, Chand G, Liu C, et al. Early phase I study of a (99m)Tc labeled anti-PD-L1 single domain antibody in SPECT/CT assessment of programmed death ligand-1 expression in non-small cell lung cancer. J Nucl Med. February 2019.
24. Rashidian M, Ploegh H. Nanobodies as non-invasive imaging tools. IOTECH. 2020.
25. Andrews LP, Marciscano AE, Drake CG, Vignali DAA. LAG3 (CD223) as a cancer immunotherapy target. Immunol Rev. 2017;276:80-96.
26. Huang C-T, Workman CJ, Flies D, et al. Role of LAG-3 in regulatory T cells. Immunity. 2004;21:503-513.
27. Versteven M, Van den Bergh JMJ, Broos K, et al. A versatile T cell-based assay to assess therapeutic antigen-specific PD-1-targeted approaches. Oncotarget. 2018;9:27797-27808.
28. Lemaire M, D'Huyvetter M, Lahoutte T, et al. Imaging and radioimmunotherapy of multiple myeloma with anti-idiotypic Nanobodies. Leukemia. 2014;28:444-447.
29. Breckpot K, Dullaers M, Bonehill A, et al. Lentivirally transduced dendritic cells as a tool for cancer immunotherapy. J Gene Med. 2003;5:654-667.
30. Yu X, Huang X, Chen X, et al. Characterization of a novel anti-human lymphocyte activation gene 3 (LAG-3) antibody for cancer immunotherapy. MAbs. June 2019:1-10.

## Claims

1. huLAG-3 VHHs consist of 4 framework regions (FR1 to FR4 respectively) and 3 complementarity determining regions (CDR1 to CDR3 respectively), in which at least one of the CDR1, CDR2 and CDR3 sequence is chosen from the group consisting of the CDR1, CDR2, CDR3 sequences respectively listed in Table 1(a-c); or from the group of CDR1, CDR2 and CDR3 sequences, respectively, that have at least 80%, preferably at least 90%, more preferably at least 95%, even more preferably at least 99% "sequence identity" (as defined herein) with at least one of the CDR1, CDR2 and CDR3 sequences, respectively, listed in Table 1(a-c)

2. huLAG-3 VHHs according to claim 1 in which the 4 framework regions (FR1 to FR4 respectively) is chosen from the group consisting of the FR1, FR2, FR3, and FR4 sequences respectively listed in Table 2(a-d); or from the group of FR1, FR2, FR3, and FR4 sequences, respectively, that have at least 80%, preferably at least 90%, more preferably at least 95%, even more preferably at least 99% "sequence identity" (as defined herein) with at least one of the FR1, FR2, FR3, and FR4 sequences, respectively, listed in Table 2(a-d).

3. huLAG-3 VHHs according to claims 1 or 2, wherein the huLAG-3 VHHs are selected from the group of VHH sequences listed in Table 2; or from VHH sequences that have at least 80%, preferably at least 90%, more preferably at least 95%, even more preferably at least 99% "sequence identity" (as defined herein) with at least one of the VHH sequences listed in Table 3 below.

4. huLAG-3 VHHs according to any one of the previous claims, wherein the huLAG-3 VHHs are selected from VHH 3150 (SEQ ID 113) or VHH 3187 (SEQ ID 119); or from VHH sequences that have at least 80%, preferably at least 90%, more preferably at least 95%, even more preferably at least 99% "sequence identity" (as defined herein) with at least one of VHH 3150 (SEQ ID 113) or VHH 3187 (SEQ ID 119).

5. huLAG-3 VHHs according to any one of the previous claims, for use as a medicine.

6. huLAG-3 VHHs according to any one of the previous claims, for use in the prevention, diagnosis and/or treatment of diseases and disorders associated with huLAG-3 expression, and in particular with diseases and disorders associated with or **characterised by** an over-expression of huLAG-3 (i.e. in certain tissues or cells). Examples of such diseases and disorders will be clear to the skilled person, and include various forms of cancers and tumors, such as those mentioned herein, as well as solid tumors (advanced and/or metatstatic), non-small-cell lung cancer, glioblastoma, melanoma, breast-, hematological-, head and neck-, liver-, gastric-, kidney-, oesophageal- and colon-cancer,
